# EUROPEAN PATENT APPLICATION

(11) **EP 3 428 647 A1**
(43) Date of publication of application: **16.01.2019**
(21) Application number: 17382457.4
(22) Date of filing: 12.07.2017
(51) Int. Cl.: G01N 33/574, A61K 31/00, C12Q 1/68

(54) **EXPRESSION SIGNATURE FOR GLIOMA DIAGNOSIS AND/OR PROGNOSIS IN A SUBJECT**

(71) Applicant: Consejo Superior de Investigaciones Científicas (CSIC), 41013 Sevilla (ES); Servicio Andaluz de Salud, 41071 Sevilla (ES); Universidad De Sevilla, 41013 Sevilla (ES)
(72) Inventor: PEINADO SERRANO, Javier, 41013 Sevilla (ES); LUCENA CACACE, Antonio, 41013 Sevilla (ES); JIMENEZ GARCIA, Manuel Pedro, 41013 Sevilla (ES); OTERO ALBIOL, Daniel, 41013 Sevilla (ES); CARNERO MOYA, Amancio, 41013 Sevilla (ES)
(74) Representative: Pons

(57) **Abstract**

The present invention relates to an *in vitro* method for diagnosing glioma in a subject, for determining the prognosis of a subject suffering from glioma, or determining the response of a subject to a therapy against glioma, comprising (a) determining the expression levels of NAMPT gene and, additionally, the expression levels of, at least, two, three, four, five, six or seven genes from the list consisting of CD44, Jun, TEAD4, CSNK1A1, ABCC3, Serpine1 and HES1 in an isolated biological sample from the subject. The invention also relates to a kit for carrying out the method of the invention and to the uses thereof.

## Description

The invention relates to a gene signature useful for diagnosing glioma in a subject, for determining the prognosis of a subject suffering from glioma, or for determining the response of a subject to a therapy against glioma. The gene signature comprises the NAMPT gene in combination with, at least, one gene from the list consisting of CD44, c-Jun, TEAD4, CSNK1A1, ABCC3, Serpine1 and HES1. The invention also relates to a kit comprising reagents for determining the expression levels of said genes.

### BACKGROUND ART

Gliomas are by far the most common primary brain tumors in adults and account for more than 50% of all brain tumors. The main types of neuroepithelial tumors are ependymomas, astrocytomas, and oligodendrogliomas, although there also exist mixed cellular forms, such as oligoastrocytomas. The World Health Organization classifies astrocytomas according to their degree of anaplasia into grade II (diffuse astrocytoma), grade III (anaplastic astrocytoma) and grade IV (glioblastoma: GBM)1. In adults, the most common tumors are high-grade neoplasms derived from astrocytes or oligodendrocytes than are diagnosed as such (primary GBM) or develop from a more benign astrocytoma (secondary GBM). Generically, high grade gliomas are highly vascular tumors and have a tendency to infiltrate tissues, create necrosis and hypoxia, and destroy the blood-brain barrier where the tumor is located. "Low grade" gliomas are typically well-differentiated, slower growing, biologically less aggressive, and portend a better prognosis for the patient; while the "high grade" gliomas, are undifferentiated or anaplastic, fast growing and can invade adjacent tissues, and carry a worse prognosis.

Symptoms of gliomas depend on which part of the central nervous system is affected. A brain glioma can cause seizures, headaches, nausea and vomiting (as a result of increased intracranial pressure), mental status disorders, sensory-motor deficits, etc. A glioma of the optic nerve can cause visual loss. Spinal cord gliomas can cause pain, weakness, numbness in the extremities, paraplegia, tetraplegia, etc. Gliomas do not metastasize by the bloodstream, but they can spread via the cerebrospinal fluid and cause "drop metastases" to the spinal cord.

The patent application US2015/0038357 discloses a method of determining the survival prognosis of a patient afflicted by a glioma. The method included assessing the level of expression of one or more specific genes selected from POSTN gene, HSPG2 gene, COL1A1 gene, NEK2 gene, DLG gene, FOXM1 gene, PLK1 gene, NKX6-1 gene, NRG3 gene, BUB1 B gene, JAG1 gene, EZH2 gene and BUB1 gene.

The patent application US2012/0129711 discloses the identification and use of biomarkers with clinical relevance to high grade gliomas. The patent application provides the identity of four genes, CHAF1 B, PDLIM4, EDNRB and HJURP, whose expression, at the transcriptome and proteome levels, is correlated with HGG grading and clinical survival outcome.

The international patent application WO2008/109423 discloses a set of genes, the expression of which has a prognostic value, specifically with respect to the survival, for example, disease-free survival and/or response to therapy.

However, none of these sets of genes relates to cancer stem cell pathways which have been associated with chemotherapy and radiotherapy resistance in glioma tumor. Therefore, they are not a reliable indicative of the response of a patient to the therapy. Thus, there is a need in the art for a diagnostic method that can more easily be established in clinical settings and provides a reliable prognosis of subject suffering from glioma.

### DETAILED DESCRIPTION OF THE INVENTION

The authors of the present invention have found that the expression levels of NAMPT gene are increased in subjects suffering from glioma. Thus, the present invention relates to a method for diagnosing glioma in a subject, for predicting the prognosis of glioma patients and/or for determining the response of a patient to a therapy against glioma.

Additionally, the authors of the present invention have also found that the expression levels of NAMPT gene are associated with other genes whose expression levels are also increased with respect to a reference value. Thus, the inventors conclude that a subject suffering from glioblastoma comprise a set of genes which are overexpressed with respect to a reference value, being said set of genes a gene signature useful for diagnosing glioma and/or for predicting the prognosis of glioma patients and/or for determining the response of a patient to a therapy against glioma. The gene signature comprises the NAMPT gene in combination with and, at least, one, two, three, four, five, six or seven genes, selected from the list consisting of CD44, Jun, TEAD4, CSNK1A1, ABCC3, Serpine1 and HES1. The authors arrived at this conclusion after analyzing the correlation between glioma patient survival and gene expression in brain tissue from patients suffering from glioblastoma (Example 1).

Thus, in one aspect, the invention relates to an *in vitro* method for diagnosing glioma in a subject, for determining the prognosis of a subject suffering from glioma, or for determining the response of a subject to a therapy against glioma, comprising
(a) determining the expression levels of NAMPT gene and, additionally, the expression levels of, at least, one, two, three, four, five, six or seven genes from the list consisting of CD44, c-Jun, TEAD4, CSNK1A1, ABCC3 and HES1 in an isolated biological sample from the subject, and
(b) comparing the expression levels obtained in step (a) with a reference value for each gene,
wherein if the expression levels of said genes are increased with respect to the reference value for each gene, then said subject suffers from glioma, said subject has a poor prognosis, or said subject is not responding to the therapy.

Thus, in another aspect, the invention relates to an *in vitro* method for diagnosing glioma in a subject, for determining the prognosis of a subject suffering from glioma, or for determining the response of a subject to a therapy against glioma, hereinafter "method of the invention", comprising
(a) determining the expression levels of NAMPT gene and, additionally, the expression levels of, at least, two, three, four, five, six or seven genes from the list consisting of CD44, c-Jun, TEAD4, CSNK1A1, ABCC3, Serpine1 and HES1 in an isolated biological sample from the subject, and
(b) comparing the expression levels obtained in step (a) with a reference value for each gene,
wherein if the expression levels of said genes are increased with respect to the reference value for each gene, then said subject suffers from glioma, said subject has a poor prognosis, or said subject is not responding to the therapy.

Thus, these genes can be used as biomarkers, hereinafter "biomarkes of the invention", for diagnosing glioma in a subject, for determining the prognosis of a subject suffering from glioma, or for determining the response of a subject to a therapy against glioma.

The term "diagnosing" refers to the identification of the nature of an illness, disease or other problem by examination of the symptoms, parameters or biomarkers in a subject. In the context of the present invention, the biomarkers are the gene signature defined in the present invention and the illness or disease is glioma.

The term "prognosis" refers to the prediction of the likelihood of cancer-attributable death or progression, including recurrence, metastatic spread and drug resistance, of a disease such as glioma. The term "prediction" is used herein to refer to the likelihood that a patient will have a particular clinical outcome. As will be explained later, the clinical outcome may be positive or negative. The predictive methods of the present invention can be used clinically to make treatment decisions by choosing the most appropriate treatment modalities for any particular patient. The predictive method of the present invention is a valuable tool in predicting if a patient is likely to respond favourably to a treatment regimen.

In the present invention "clinical outcome" is understood as the expected course of a disease. It denotes the doctor's prediction of how a subject's disease will progress, and whether there is chance of recovery o recurrence.

The term "negative clinical outcome" or "poor prognosis" means a decreased in any measure of patient status, including those measures ordinarily used in the art, such as the duration of Recurrence-Free interval (RFI), the time of Overall Survival (OS), the time of Disease-Free Survival (DFS), the duration of Distant Recurrence-Free Interval (DRFI), and the like. An increase in the likelihood of positive clinical outcome corresponds to a decrease in the likelihood of cancer recurrence.

The prediction of the clinical outcome can be done by using any endpoint measurements used in oncology and known to the skilled practitioner. Useful endpoint parameters to describe the evolution of a disease include:
▪ disease-free progression which, as used herein, describes the proportion of subjects in complete remission who have had no recurrence of disease during the time period under study;
▪ objective response, which, as used in the present invention, describes the proportion of treated people in whom a complete or partial response is observed;
▪ tumor control, which, as used in the present invention, relates to the proportion of treated people in whom complete response, partial response, minor response or stable disease ≥ 6 months is observed.
▪ progression free survival which, as used herein, is defined as the time from start of treatment to the first measurement of cancer growth.
▪ six-month progression free survival or "PFS6" rate which, as used herein, relates to the percentage of people wherein free of progression in the first six months after the initiation of the therapy
▪ "disease-free survival" (DFS) is understood as the length of time after treatment for a specific disease during which a subject survives with no sign of the disease, and
▪ median survival which, as used herein, relates to the time at which half of the subjects enrolled in the study.

In a particular embodiment, the clinical outcome is measured as overall survival.

In the present invention, the "overall survival" (also called OS) refers to the length of time from either the date of diagnosis or the start of treatment for a disease, such as cancer, that patients diagnosed with the disease are still alive. In a clinical trial, measuring the overall survival is one way to see how well a new treatment works.

The calculation of the measures listed above in practice may vary from study to study depending on the definition of events to be either censored or not considered. The expression "determining the response of a subject to a therapy" refers to the assessment of the results of a therapy in a patient with glioma in response to a therapy. The response of the subject to the therapy may be negative (the subject does not respond to the therapy) or positive (the subject responds to the therapy).

In the present invention, it is considered that a subject has a "negative response to the therapy" or "does not response to the therapy" when, during and immediately after treatment, the decrease in size of the tumour is less than 25%, the tumour is stabilized or the tumour keep growing unaffected by the therapy. On the contrary, it is considered that a subject has a "positive response to the therapy" or "does response to the therapy" when the decrease in size of the tumour is more than 25% or the tumour is undetectable. A negative response of the subject to the therapy means that the therapy administered is not being effective. A high number of treatments are available for addressing glioma and any of them can be used in the context of the present invention. An example of glioma treatments include, without limiting to, the therapies listed in Table 1 below.

**Table 1: List of therapies which can be administered to a subject for treating glioma.**

| **Therapy** |
|---|
| Monoclonal Antibody Conjugate to Target B7H3 for Pediatric Diffuse Intrinsic Pontine Glioma |
| Cellular Immunotherapy to Target EGFR for Glioma |
| FLAG-003 |
| Monoclonal Antibodies to Antagonize Hepatocyte Growth Factor Receptor for Glioma |
| PolyPEPI-1112 |
| Small Molecule to Activate PPAR Gamma for Glioma |
| Small Molecules to Agonize Cannabinoid Receptor for Glioma |
| Cellular Immunotherapy to Target EPHA3 for Glioma |
| Monoclonal Antibody to Antagonize the Tenascin for Glioma |
| ranaigengliotucel-T |
| Cellular Immunotherapy to Target EphA2 for Recurrent Malignant Glioma |
| Cellular Immunotherapy to Target IDH1 for Glioma |
| Monoclonal Antibody Conjugate to Antagonize EGFR for Brain Tumor, Malignant Glioma and Glioblastoma Multiforme |
| Vaccine to Target URLC10, DEPDC1, FOXM1, KIF20A VEGFR1 and VEGFR2 for Malignant Glioma |
| Cellular Immunotherapy to Target IL-13 for Malignant Glioma |
| 7-A2 |
| Vaccine to Target OX40 Ligand for Glioma |
| Cellular Immunotherapy to Target CD3 and CD56 for Malignant Glioma |
| Cellular Immunotherapy to Target IL-13R for Malignanat Glioma |
| Gene Therapy for High-Grade Glioma and Liver Transplantation |
| Gene Therapy to Activate Alpha-2,6-Sialyltransferase for Malignant Glioma |
| Oligonucleotides to Inhibit MMP-2 and MMP-9 for Glioma |
| Synthetic Peptide to Antagonize Neuropilin-1 Receptor for Glioma |
| bevacizumab |
| pemetrexed disodium |
| erlotinib hydrochloride |
| sorafenib tosylate |
| bendamustine hydrochloride |
| panitumumab |
| pembrolizumab |
| dabrafenib mesylate + trametinib dimethyl sulfoxide |
| cabazitaxel |
| temozolomide |
| lenvatinib mesylate |
| tranilast |
| irinotecan hydrochloride |
| afatinib dimaleate |
| apatinib |
| arsenic trioxide |
| bleomycin sulfate |
| carmustine |
| dabrafenib mesylate |
| DCVax-L |
| dianhydrogalactitol |
| doxorubicin CR |
| ipilimumab + nivolumab |
| irinotecan hydrochloride CR |
| leflunomide |
| nimotuzumab |
| nintedanib |
| panobinostat |
| porfimer sodium |
| procarbazine hydrochloride |
| talaporfin sodium |
| tamoxifen |
| tamoxifen citrate |
| temsirolimus |
| teniposide |
| Vaccine to Target Heat Shock Protein qp96 for Oncology |
| vandetanib |
| vincristine sulfate |
| vitespen |
| vorinostat |
| enasidenib mesylate |
| etirinotecan pegol |
| AZD-4547 |
| cannabidiol |
| crenolanib besylate |
| dacomitinib |
| depatuxizumab mafodotin |
| eflornithine hydrochloride |
| flucytosine ER + vocimagene amiretrorepvec |
| galunisertib |
| ivosidenib |
| larotrectinib |
| paclitaxel trevatide |
| pegargiminase |
| racotumomab |
| Recombinant Protein for Oncology |
| selinexor |
| selumetinib sulfate |
| (A-10 + AS-21) |
| 2X-111 |
| 2X-131 |
| ABTL-0812 |
| AdRTSIL-12 |
| alisertib |
| aminolevulinic acid hydrochloride |
| asinercept |
| ATL-101 |
| AZD-1775 |
| BMX-001 |
| Carboxyamidotriazole Orotate |
| cavatak |
| CDX-1401 |
| Cellular Immunotherapy for Oncology |
| Cellular Immunotherapy to Target MUC1 for Oncology |
| Cellular Immunotherapy to Target MUC1 for Solid Tumors |
| CYT-107 |
| depatuxizumab |
| DNX-2401 |
| DSP-7888 |
| flucytosine + TBio-01 |
| fresolimumab |
| GliAtak |
| I131-CLR1404 |
| IMA-950 |
| indoximod |
| irofulven |
| LP-561A1 |
| marizomib |
| MDNA-55 |
| milciclib |
| MK-2206 |
| NRCAN-019 |
| Parvoryx |
| pelareorep |
| pidilizumab |
| pritumumab |
| RRX-001 |
| SL-701 |
| SPM-011 |
| Teleukin |
| TVI-Brain-1 |
| USL-311 |
| verubulin |
| AG-881 |
| ALD-451 |
| BAY-1436032 |
| bevacizumab biosimilar |
| BG-00001 |
| CBT-101 |
| Cellular Immunotherapy + edodekin alfa |
| chloroquine |
| DCC-3014 |
| disufenton sodium |
| G-207 |
| GDC-0084 |
| HSV-1716 |
| I-Tenatumomab |
| IDH-305 |
| irinotecan hydrochloride + TBio-02 |
| Kid EDV |
| KX-02 |
| mibefradil dihydrochloride |
| MTX-110 |
| ONO-7579 |
| p28 |
| PAC-1 |
| PF-06840003 |
| VIMO-001 |
| WP-1066 |
| 105-F |
| 3E-10 |
| A-1R |
| ABT-737 |
| ALM-301 |
| AMG-511 |
| Angiocidin |
| BACPT-DP |
| BVA-501 |
| Cellaxane |
| cilengitide |
| CM-118 |
| doxorubicin |
| GB-13 |
| GLN-1001 |
| IDD-004 |
| Imx-110 |
| Imx-111 |
| JS-K |
| NEO-214 |
| NMHSS-1 |
| OGD-201 |
| Recombinant Peptide to Target TPI-1 and GRP-78 for Oncology |
| SENL-005 |
| Trilexium |
| z-90099 |
| zoledronic acid |
| AZD-7451 |
| BSI-018 |
| DT-010991 |
| MTX-111 |
| ORX-101 |
| TM-601 |
| AC CJ-001 |
| AC-03636 |
| Ac-929C |
| alanosine |
| ANG-1490 |
| Ang-797 |
| ATN-161 |
| BAY-846 |
| BC-5000s |
| BCL-003 |
| berubicin hydrochloride |
| carmustine SR |
| CC-8490 |
| cintredekin besudotox |
| cositecan |
| CPC-410 |
| CT-3610 |
| DP-6865 |
| EG-012 |
| Eir-060 |
| EMD-55900 |
| ETS-2101 |
| foretinib |
| gimatecan |
| Haw mAb-11 |
| HuL-2G7 |
| IMA-102 |
| iodine I 131 derlotuximab biotin |
| KRX-0402 |
| laromustine |
| LD-224 |
| litenimod |
| Monoclonal Antibody to Inhibit Receptor Tyrosine Phosphatase for Oncology |
| Monoclonal Antibody to Inhibit Tyrosine Kinase for Oncology |
| MSM-608 |
| MTS-01 |
| NC-3 |
| Neuradiab |
| OCM-124 |
| Oncolytic Virus for Oncology |
| OSIP-486823 |
| paclitaxel |
| PB-4 |
| perifosine |
| PTX-001 |
| PTX-004 |
| QC-56 |
| ridaforolimus |
| rilotumumab |
| RNAi Oligonucleotide to Inhibit VEGFR2 for Oncology and Ophthalmology |
| sitimagene ceradenovec |
| Small Molecules for Glioblastoma Multiforme |
| Small Molecules to Inhibit Glycolytic Enzymes for Cancer |
| sodium phenylbutyrate |
| talampanel |
| tandutinib |
| Tenarad |
| terameprocol |
| TLK-58747 |
| Toca-Gamma |
| Toca-RNAi |
| topotecan hydrochloride |
| UNBS-1450 |
| vatalanib |
| VB-6011 |
| VIMRxyn |
| voxtalisib |
| XL-541 |
| AC-480 |
| iniparib |
| OGX-225 |
| TransMID |
| Cellular Immunotherapy to Target HER2 for Oncology |
| Cellular Immunotherapy to Target Wilm's Tumor 1 for Oncology |
| Vaccine to Target CD155/NECL5 for Recurrent Glioblastoma Multiforme |
| Vaccine to Target VEGFR1 and VEGFR2 for Oncology |
| Vaccine to Target Wilm's Tumor 1 for Oncology |
| Cellular Immunotherapy for Gliomas |
| Cellular Immunotherapy to Target MAGE-A1, MAGE-A3 and NY-ESO-1 for Oncology |
| CIGB-128 |
| Vaccine to Target WT1 for Oncology |
| Monoclonal Antibodies to Inhibit Pleiotrophin for Pancreatic and Breast Cancer |
| Protein for Oncology, Ophthalmology and Metabolic Disorders |
| Recombinant Proteins to Target Interleukin-13 for Glioblastoma Multiforme |
| rQNestin-34.5v.2 |
| SH-454 |
| SH-507 |
| Small Molecule to Agonize GPR55 for Oncology |
| Small Molecules to Target Beta 2 Adrenergic Receptor and Cannabinoid Receptor for Oncology and Congestive Heart Failure |
| Tatbeclin-1 |
| TRA-8 |
| Vaccine for Glioblastoma Multiforme |
| Antibody to Inhibit Tenascin-C for Chronic Inflammation |
| aurintricarboxylic acid |
| dorsomorphin |
| EDL-358 |
| Recombinant Peptide to Inhibit EGFR for Oncology |
| Small Molecules to Inhibit GLI1 for Breast Cancer |
| SP-627 |
| Xaripin D |
| Xaripin P |
| Antibody to Inhibit Granulin D for Oncology |
| Monoclonal Antibody Conjugate to Inhibit Chondroitin Sulfate Proteoglycan for Oncology |
| Rotem-201 |
| sarmustine |
| Small Molecule for Brain Cancer |
| tetrandrine |

Nevertheless, in a particular embodiment, the therapy against glioma is selected from the group consisting of a NAMPT inhibitor, an angiogenesis inhibitor, preferably an VEGF inhibitor, Opdivo, Rintega, Temozolomide (TMZ), ICT-107, Avastin, ABT-414 in combination with TMZ, ITK-1, Toca511 in combination with Toca-FC, Avastin in combination with VB-111, Veliparib in combination with TMZ, DCVax-L in combination with TMZ, and combinations thereof.

In another particular embodiment, the NAMPT inhibitor is selected from the group consisting of FK866, CHS828, GNE-617, OT-82, MPC-8640, MPC-9528 and combinations thereof, such as FK866 and CHS828; FK866 and GNE-617; FK866 and OT-82; FK866 and MPC-8640; FK866 and MPC-9528; CHS828 and GNE-617; CHS828 and OT-82; CHS828 and MPC-8640; CHS828 and MPC-9528; GNE-617 and OT-82; GNE-617 and MPC-8640; GNE-617 and MPC-9528; OT-82 and MPC-8640; OT-82 and MPC-9528; MPC-8640 and MPC-9528; FK866, CHS828 and GNE-617; FK866, CHS828 and OT-82; FK866, CHS828 and MPC-8640; FK866, CHS828 and MPC-9528; FK866, CHS828, GNE-617 and OT-82; FK866, CHS828, GNE-617 and MPC-8640; FK866, CHS828, GNE-617 and MPC-9528; FK866, CHS828, GNE-617, OT-82 and MPC-8640; FK866, CHS828, GNE-617, OT-82 and MPC-9528; FK866, CHS828, GNE-617, OT-82, MPC-8640 and MPC-9528; CHS828, GNE-617 and OT-82; CHS828, GNE-617 and MPC-8640; CHS828, GNE-617 and MPC-9528; GNE-617, OT-82 and MPC-8640; GNE-617, OT-82 and MPC-9528; and OT-82, MPC-8640 and MPC-9528.

The use of the gene signature or biomarkers of the invention to monitor the efficacy of a treatment or therapy can also be applied to methods for selecting and screening drugs with potential anti-tumor activity. This process comprises a) administrating to the subject (preferably an animal) the drug to be studied; b) taking biological samples from the animal at different points of the study (before, during and/or after administration) and determining the levels of the biomarkers according to the present invention; and c) comparing the determinations made in the samples obtained from the subject with a reference sample.

As explained above, the method of the invention can be used for diagnosing glioma in a subject, for determining the prognosis of a subject suffering from glioma, or for determining the response of a subject to a therapy against glioma.

In the context of the present invention, the term "glioma" refers to tumors that originate from brain or spinal cord, in particular from glial cells or their progenitors. Gliomas are classified by cell type and by grade.

Gliomas are named according to the specific type of cell with which they share histological features, but not necessarily from which they originate. The main types of gliomas are Ependymomas (ependymal cells), astrocytomas (glioblastoma multiforme is a malignant astrocytoma and the most common primary brain tumor among adults), oligodendrogliomas (oligodendrocytes), brainstem glioma (developed in the brain stem), optic nerve glioma (developed in or around the optic nerve) and mixed gliomas, such as oligoastrocytomas, contain cells from different types of glia. Examples of gliomas include, but not limited to, astrocytoma, chordoma, CNS lymphoma, craniopharyngioma, medulloblastoma, meningioma, metastatic brain tumors, oligodendroglioma and schwannoma.

Gliomas are further categorized according to their grade, which is determined by pathologic evaluation of the tumor. The World Health Organization (WHO) classification system categorizes gliomas from grade I (lowest grade) through grade IV (highest grade), based upon histopathologic characteristics such as cytological atypia, anaplasia, mitotic activity, microvascular proliferation, and necrosis. Primary brain tumors are tumors that are classified by a pathologist according to their appearance under the microscope. Thus, gliomas are classified into four grades (I, II, III and IV) and the treatment and prognosis depend upon the tumor grade.
▪ Grade I or II tumors are termed low-grade gliomas.
   Low-grade gliomas (LGGs) consist of grade I tumors, which contain none of the aforementioned histologic features, and grade II tumors, characterized by the presence of cytologic atypia alone. Low-grade astrocytic tumors include diffuse astrocytoma, pilomyxoid astrocytoma, and pleomorphic xanthoastrocytoma (WHO grade II), as well as subependymal giant cell astrocytoma (SEGA) and pilocytic astrocytoma (WHO grade I tumors). Low-grade oligodendroglial tumors include oligodendrogliomas and oligoastrocytomas (WHO grade II tumors). Low-grade glioneuronal tumors include the following WHO grade I tumors: ganglioglioma, desmoplastic infantile astrocytoma and ganglioglioma, dysembryoplastic neuroepithelial tumor, papillary glioneuronal tumor, and rosette-forming glioneuronal tumor of the fourth ventricle.
▪ Grade III (anaplastic astrocytoma, anaplastic oligodendroglioma, anaplastic oligoastrocytoma, anaplastic ependymoma)
▪ Grade IV (glioblastoma)

The term malignant or high-grade glioma refers to tumors that are classified as Grade III or Grade IV.

Any kind of glioma can be diagnosed or prognosticated with the method of the invention. In a particular embodiment, the glioma is selected from the list consisting of grade I, grade II, grade III and grade IV glioma.

The method of the invention requires a first step comprising determining the expression levels of NAMPT gene and, additionally, the expression levels of, at least, two, three, four, five, six or seven genes from the list consisting of CD44, c-Jun, TEAD4, CSNK1A1, ABCC3, Serpine1 and HES1 in an isolated biological sample from the subject.

The NAMPT gene (GenBank accession number: KJ898163.1) refers to the gene encoding the nicotinamide phosphoribosyl transferase protein which catalyzes the conversion of nicotinamide to nicotinamide mononucleotide (EC 2.4.2.12). Other names which can be used in the literature for referring to this gene are, without limiting to, VF, PBEF, PBEF1, VISFATIN and 1110035O14Rik. In a particular embodiment, the NAMPT gene comprises a nucleotide sequence comprising a sequence identity of, at least, 50, 55, 60, 65, 70, 75, 80, 85, 90, 95, 96, 97, 98 or 99 % with the nucleotide sequence SEQ ID NO: 1 (NCBI reference Sequence NM_005746.2.). Thus, allelic variants of the SEQ ID NO: 1 are encompassed within the context of the present invention. Nevertheless, in a more particular embodiment, the NAMPT gene comprises the sequence SEQ ID NO: 1.

The CD44 gene (GenBank accession number: X62739.1) refers to the gene encoding CD44 protein. CD44 is the main cell surface receptor for hyaluronate. Mature lymphocytes in the circulation migrate selectively from the bloodstream to different lymphatic tissues through specialized high endothelial venules (HEV). Molecules on the surface of lymphocytes called homing receptors interact specifically with HEV and play a central role in the migration. Other names which can be used in the literature for referring to this gene include, without limiting to, CD44 ANTIGEN; CD44, HERMES ANTIGEN, Pgp1, MDU3 and INLU-RELATED p80 GLYCOPROTEIN. In a particular embodiment, the CD44 gene comprises a nucleotide sequence comprising a sequence identity of, at least, 50, 60, 65, 70, 75, 80, 85, 90, 95, 96, 97, 98 or 99 % with the nucleotide sequence SEQ ID NO: 2 (GenBank accession number: FJ216964.1). Thus, allelic variants of the SEQ ID NO: 2 are encompassed within the context of the present invention. Nevertheless, in a more particular embodiment, the CD44 gene comprises the sequence SEQ ID NO: 2.

The c-Jun gene (NCBI Reference Sequence: NM_002228.3) refers to the gene encoding V-JUN AVIAN SARCOMA VIRUS 17 ONCOGENE HOMOLOG; JUN protein which is essential for neuronal microtubule assembly and apoptosis. Phosphorylation of the activating protein 1 (AP1) transcription factor c-Jun, at multiple sites within its transactivation domain, is required for JNK-induced neurotoxicity. Other names which can be used in the literature for referring to this gene are, without limiting to, JUN, c-Jun, ACTIVATOR PROTEIN 1, AP1, ENHANCER-BINDING PROTEIN AP1. The nucleotide sequence encoding the human JUN protein comprises the sequence SEQ ID NO: 3 (NCBI Reference Sequence NM_002228.3). In a particular embodiment, the Jun gene comprises a nucleotide sequence comprising a sequence identity of, at least, 50, 60, 65, 70, 75, 80, 85, 90, 95, 96, 97, 98 or 99 % with the nucleotide sequence SEQ ID NO: 3. Thus, allelic variants of the SEQ ID NO: 3 are encompassed within the context of the present invention. Nevertheless, in a more particular embodiment, the Jun gene comprises the sequence SEQ ID NO: 3.

The TEAD4 gene (GenBank accession number: KJ897651.1) refers to the gene encoding TEAD4 protein which is related to the transcription factor TEF1 (TCF13; 189967) which directs muscle-specific gene expression. Other names which can be used in the literature for referring to this gene include, without limiting to, TEA DOMAIN FAMILY MEMBER 4; TEAD4 and TRANSCRIPTION FACTOR 13-LIKE 1; TCF13L1; TRANSCRIPTIONAL ENHANCER FACTOR 1-RELATED GENE; RTEF1; TRANSCRIPTIONAL ENHANCER FACTOR 3; TEF3. In a particular embodiment, the TEAD4 gene comprises a nucleotide sequence comprising a sequence identity of, at least, 50, 60, 65, 70, 75, 80, 85, 90, 95, 96, 97, 98 or 99 % with the nucleotide sequence SEQ ID NO: 4 (KJ897651.1). Thus, allelic variants of the SEQ ID NO: 4 are encompassed within the context of the present invention. Nevertheless, in a more particular embodiment, the TEAD4 gene comprises the sequence SEQ ID NO: 4.

The CSNK1A1 gene (GenBank accession number: KJ896661.1) refers to the gene encoding CASEIN KINASE I, ALPHA-1; CSNK1A1 protein which regulates Smo (601500) cell surface accumulation and activity in response to hedgehog (Hh; see 600725). Other names which can be used in the literature for referring to this gene include, without limiting to, CASEIN KINASE I ALPHA-1; CSNK1A1 and CK1-ALPHA CK1. In a particular embodiment, the CSNK1A1 gene comprises a nucleotide sequence comprising a sequence identity of, at least, 50, 60, 65, 70, 75, 80, 85, 90, 95, 96, 97, 98 or 99 % with the nucleotide sequence SEQ ID NO: 5 (KJ896661.1). Thus, allelic variants of the SEQ ID NO: 5 are encompassed within the context of the present invention. Nevertheless, in a more particular embodiment, the CSNK1A1 gene comprises the sequence SEQ ID NO: 5.

The ABCC3 gene (GenBank accession number: KJ892604.1) refers to the gene encoding ATP-BINDING CASSETTE, SUBFAMILY C, MEMBER 3; ABCC3 protein. Bile secretion in liver is driven in large part by ATP-binding cassette (ABC)-type proteins that reside in the canalicular membrane and effect ATP-dependent transport of bile acids, phospholipids, and non-bile acid organic anions. Other names which can be used in the literature for referring to this gene include, without limiting to, ATP-BINDING CASSETTE, SUBFAMILY C, MEMBER 3; ABCC3 and MULTIDRUG RESISTANCE-ASSOCIATED PROTEIN 3; MRP3 CANALICULAR MULTISPECIFIC ORGANIC ANION TRANSPORTER 2; CMOAT2. In a particular embodiment, the ABCC3 gene comprises a nucleotide sequence comprising a sequence identity of, at least, 50, 60, 65, 70, 75, 80, 85, 90, 95, 96, 97, 98 or 99 % with the nucleotide sequence SEQ ID NO: 6 (KJ892604.1). Thus, allelic variants of the SEQ ID NO: 6 are encompassed within the context of the present invention. Nevertheless, in a more particular embodiment, the ABCC3 gene comprises the sequence SEQ ID NO: 6.

The Serpine1 gene (GenBank accession number: KJ897292.1) refers to the gene encoding SERPIN PEPTIDASE INHIBITOR, CLADE E (NEXIN, PLASMINOGEN ACTIVATOR INHIBITOR TYPE 1), MEMBER 1; SERPINE1 protein, a member of the serine protease inhibitor family that inhibits tissue-type plasminogen activator (PLAT; 173370) and urokinase-type plasminogen activator (PLAU; 191840). PLAT and PLAU proteolytically activate plasminogen (PLG; 173350) into plasmin, which breaks down fibrin clots. Thus, SERPINE1 negatively regulates fibrinolysis and impairs the dissolution of clots. Other names which can be used in the literature for referring to this gene include, without limiting to, SERPIN PEPTIDASE INHIBITOR, CLADE E (NEXIN, PLASMINOGEN ACTIVATOR INHIBITOR TYPE 1), MEMBER 1; SERPINE1 and PLASMINOGEN ACTIVATOR INHIBITOR 1; PAl1 ENDOTHELIAL PLASMINOGEN ACTIVATOR INHIBITOR. In a particular embodiment, the Serpine1 gene comprises a nucleotide sequence comprising a sequence identity of, at least, 50, 60, 65, 70, 75, 80, 85, 90, 95, 96, 97, 98 or 99 % with the nucleotide sequence SEQ ID NO: 7 (KJ897292.1). Thus, allelic variants of the SEQ ID NO: 7 are encompassed within the context of the present invention. Nevertheless, in a more particular embodiment, the Serpine1 gene comprises the sequence SEQ ID NO: 7.

The HES1 gene (GenBank accession number: Y07572.1) refers to the gene encoding DROSOPHILA HOMOLOG OF HAIRY/ENHANCER OF SPLIT1; HES1 protein HES1 belongs to a family of basic helix-loop-helix (bHLH) proteins that are essential for neurogenesis, myogenesis, hematopoiesis, and sex determination. HES1 is a transcriptional repressor for a number of genes, but it can also function as a transcriptional activator. Other names which can be used in the literature for referring to this gene include, without limiting to, DROSOPHILA HOMOLOG OF HAIRY, and HRY. In a particular embodiment, the HES1 gene comprises a nucleotide sequence comprising a sequence identity of, at least, 50, 60, 65, 70, 75, 80, 85, 90, 95, 96, 97, 98 or 99 % with the nucleotide sequence SEQ ID NO: 8 (Y07572.1). Thus, allelic variants of the SEQ ID NO: 8 are encompassed within the context of the present invention. Nevertheless, in a more particular embodiment, the HES1 gene comprises the sequence SEQ ID NO: 8.

The term "allelic variant" means any of two or more alternative forms of a gene occupying the same chromosomal locus. Allelic variation arises naturally through mutation, and may result in polymorphism within populations. Gene mutations can be silent (no change in the encoded polypeptide) or may encode polypeptides having altered amino acid sequences. An allelic variant of a polypeptide is a polypeptide encoded by an allelic variant of a gene.

In the present invention, "identity" or "sequence identity" is understood to mean the degree of similarity between two nucleotide or amino acid sequences obtained by aligning the two sequences. Depending on the number of common residues between the aligned sequences, a different degree of identity, expressed as a percentage, will be obtained. Nucleotide sequences are said to be homologous when exhibiting a certain degree of identity. The degree of identity between two amino acid sequences may be determined by conventional methods, for example, by standard sequence alignment algorithms known in the state of the art, such as, for example, BLAST [Altschul S.F. et al. Basic local alignment search tool. J Mol Biol. 1990 Oct 5; 215(3): 403-10]. The BLAST programmes, for example, BLASTN, BLASTX, and T BLASTX, BLASTP and TBLASTN, are in the public domain at *The National Center for Biotechonology Information* (NCBI) website. The skilled person in the art understands that mutations in the nucleotide sequence of genes that lead to conservative amino acid substitutions at non-critical positions for the functionality of the protein are evolutionarily neutral mutations which do not affect its global structure or its functionality.

In performing the method of the present invention, a sample is obtained from the subject. The term "sample" as used herein, relates to any sample which can be obtained and isolated from the subject.

The term "isolated" means a substance in a form or environment that does not occur in nature. Non-limiting examples of isolated substances include (1) any non-naturally occurring substance, (2) any substance including, but not limited to, any enzyme, variant, nucleic acid, protein, peptide or cofactor, that is at least partially removed from one or more or all of the naturally occurring constituents with which it is associated in nature; (3) any substance modified by the hand of man relative to that substance found in nature; or (4) any substance modified by increasing the amount of the substance relative to other components with which it is naturally associated.

The present method can be applied to any type of biological sample from a subject, such as a biopsy sample, tissue (e.g. brain tissue), cell or fluid (serum, saliva, semen, sputum, cerebral spinal fluid (CSF), tears, mucus, sweat, brain extracts and the like). In a particular embodiment, said sample is a brain tissue sample or portion thereof, blood or serum.

A tissue sample can be obtained by conventional methods, e.g., biopsy, by using methods well known to those of ordinary skill in the related medical arts. Methods for obtaining the sample from the biopsy include gross apportioning of a mass, or microdissection or other art-known cell-separation methods. In order to simplify conservation and handling of the samples, these can be formalin-fixed and paraffin-embedded or first frozen and then embedded in a cryosolidifiable medium, such as OCT-Compound, through immersion in a highly cryogenic medium that allows for rapid freeze.

The method of the invention requires determining the expression levels of NAMPT gene and, additionally, the expression levels of, at least, two, three, four, five, six or seven genes from the list consisting of CD44, Jun, TEAD4, CSNK1A1, ABCC3, Serpine1 and HES1 in an isolated biological sample from a subject.

The term "expression" includes any step involved in the production of a polypeptide including, but not limited to, transcription, post-transcriptional modification, translation, post-translational modification, and secretion. Therefore, as the person skilled in the art understands, the expression levels of the genes can be measured by determining the mRNA expression levels of said genes or by determining the protein levels encoded by said genes.

In a particular embodiment, determining the gene expression levels comprises quantifying the messenger RNA (mRNA) of said genes, or a fragment of said mRNA, the complementary DNA (cDNA) of said genes, or a fragment of said cDNA.

In order to measure the mRNA levels of biomarkers of the invention, the biological sample may be treated to physically or mechanically disrupt tissue or cell structure, to release intracellular components into an aqueous or organic solution to prepare nucleic acids for further analysis. The nucleic acids are extracted from the sample by procedures known to the skilled person using commercially available reagents. RNA is then extracted from frozen or fresh samples by any of the methods typical in the art [Sambrook, Fischer and Maniatis, Molecular Cloning, a laboratory manual, (2nd ed.), Cold Spring Harbor Laboratory Press, New York, (1989)]. Preferably, care is taken to avoid degradation of the RNA during the extraction process.

The expression level can be determined using mRNA obtained from a formalin-fixed, paraffin-embedded tissue sample coming from a subject as defined above. In this case, the tissue sample is first deparaffinized. An exemplary deparaffinization method involves washing the paraffinized sample with an organic solvent, such as xylene, for example. Deparaffinized samples can be rehydrated with an aqueous solution of a lower alcohol. Suitable lower alcohols, for example include, methanol, ethanol, propanols, and butanols. Deparaffinized samples may be rehydrated with successive washes with lower alcoholic solutions of decreasing concentration, for example. Alternatively, the sample is simultaneously deparaffinized and rehydrated. The sample is then lysed and RNA is extracted from the sample.

While all techniques of gene expression profiling (Real Time-PCR, SAGE, or TaqMan®) are suitable for use in performing the foregoing aspects of the invention, the mRNA expression levels are often determined by reverse transcription polymerase chain reaction (RT-PCR). The detection can be carried out in individual samples or in tissue microarrays.

In a particular embodiment, the expression levels of the genes are determined by quantitative polymerase chain reaction (QPCR), Real-Time PCR (RT-qPCR), Retro-Transcriptase polymerase chain reaction (RT-PCR), a DNA array, a, RNA array or nucleotide hybridization technique.

As can be seen in the examples illustrative of the present invention, total cellular RNA was isolated with the RNeasy kit (Qiagen) and reversed transcribed into cDNA using 2.0 µg of RNA, random primers, dNTP mix and a Multiscribe Reverse Transcriptase in a total volume of 50 µL (High Capacity Transcription Kit-Applied Biosystems). Real time PCR was performed on an Applied Biosystem 7900HT cycler using gene-specific probes from Life technologies as follows: NAMPT (Hs00237184_m1), SNAI1 (Hs00195591_m1), TWIST1 (Hs01675818_s1), FOXC2 (Hs00270951_s1), VIM (Hs00185584m1), NANOG (Hs04260366_g1), BMI-1 (Hs00995536_m1), SOX2 (Hs01053049_s1), OCT4 (Hs00999632_g1), SERPINE1 (Hs00167155_m1), CD133 (Hs01009257_m1), ABCC3 (Hs00978452_m1), CSNK1A1 (Hs00793391_m1), HES1 (Hs00172878_m1), TEAD4 (Hs01125032_m1), JUN (Hs01103582_s1), CD44 (Hs01075864_m1) and GADPH (Hs03929097_g1) as housekeeping. Relative quantitation values were expressed as 2-ÄCt or relative mRNA expression.

In order to normalize the values of mRNA expression among the different samples, it is possible to compare the expression levels of the mRNA of interest in the test samples with the expression of a control RNA. A "Control RNA" as used herein, relates to a RNA whose expression levels do not change or change only in limited amounts in tumor cells with respect to non-tumorigenic cells. Preferably, the control RNA are mRNA derived from housekeeping genes and which code for proteins which are constitutively expressed and carry out essential cellular functions. Examples of housekeeping genes for use in the present invention include β-2-microglobulin, ubiquitin, 18-S ribosomal protein, cyclophilin, GAPDH and β-action. In a particular embodiment, the control RNA is β-action mRNA. In one embodiment, relative gene expression quantification is calculated according to the comparative Ct method using β-actin as an endogenous control and commercial RNA controls as calibrators. Final results, are determined according to the formula 2-(ΔCt sample-ΔCt calibrator), where ΔCT values of the calibrator and sample are determined by subtracting the CT value of the target gene from the value of the β-actin gene

In another particular embodiment, the expression level of said genes comprises quantifying the levels of protein encoded by said genes or of a functionally equivalent variant of said proteins.

In a particular embodiment, the amino acid sequence of the protein encoded by the NAMPT gene comprises the sequence SEQ ID NO: 9.
SEQ ID NO: 9:

In another particular embodiment, the amino acid sequence of the protein encoded by the CD44 gene comprises the sequence SEQ ID NO: 10.
SEQ ID NO: 10

MDKFWWHAAWGLCLVPLSLAQIDLNITCRFAGVFHVEKNGRYSISRTEAADLCKAFNST
LPTMAQMEKALSIGFETCRYGFIEGHVVIPRIHPNSICAANNTGVYILTSNTSQYDTYC
FNASAPPEEDCTSVTDLPNAFDGPITITIVNRDGTRYVQKGEYRTNPEDIYPSNPTDDD
VSSGSSSERSSTSGGYIFYTFSTVHPIPDEDSPWITDSTDRIPATRHSHGSQEGGANTT
SGPIRTPQIPEWLIIILASLLALALILAVCIAVNSRRRCGQKKKLVINSGNGAVEDRKPS
GLNGEASKSQEMVHLVNKESSETPDQFMTADETRNLQNVDMKIGV

In another particular embodiment, the amino acid sequence of the protein encoded by the c-Jun gene comprises the sequence SEQ ID NO: 11.
SEQ ID NO: 11

MTAKMETTFYDDALNASFLPSESGPYGYSNPKILKQSMTLNLADPVGSLKPHLRAKNSD
LLTSPDVGLLKLASPELERLIIQSSNGHITTTPTPTQFLCPKNVTDEQEGFAEGFVRAL
AELHSQNTLPSVTSAAQPVNGAGMVAPAVASVAGGSGSGGFSASLHSEPPVYANLSNFN
PGALSSGGGAPSYGAAGLAFPAQPQQQQQPPHHLPQQMPVQHPRLQALKEEPQTVPEMP
GETPPLSPIDMESQERIKAERKRMRNRIAASKCRKRKLERIARLEEKVKTLKAQNSELA
STANMLREQVAQLKQKVMNHVNSGCQLMLTQQLQTF

In another particular embodiment, the amino acid sequence of the protein encoded by the TEAD4 gene comprises the sequence SEQ ID NO: 12.
SEQ ID NO: 12

MYGRNELIARYIKLRTGKTRTRKQVSSHIQVLARRKAREIQAKLKDQAAKDKALQSMAA
MSSAQIISATAFHSSMALARGPGRPAVSGFWQGALPGQAGTSHDVKPFSQQTYAVQPPL
PLPGFESPAGPAPSPSAPPAPPWQGRSVASSKLWMLEFSAFLEQQQDPDTYNKHLFVHI
GQSSPSYSDPYLEAVDIRQIYDKFPEKKGGLKDLFERGPSNAFFLVKFWADLNTNIEDE
GSSFYGVSSQYESPENMIITCSTKVCSFGKQVVEKVETEYARYENGHYSYRIHRSPLCE
YMINFIHKLKHLPEKYMMNSVLENFTILQVVTNRDTQETLLCIAYVFEVSASEHGAQHH
IYRLVKE

In another particular embodiment, the amino acid sequence of the protein encoded by the CSNK1A1 gene comprises the sequence SEQ ID NO: 13.
SEQ ID NO: 13

MASSSGSKAEFIVGGKYKLVRKIGSGSFGDIYLAINITNGEEVAVKLESQKARHPQLLY
ESKLYKILQGGVGIPHIRWYGQEKDYNVLVMDLLGPSLEDLFNFCSRRFTMKTVLMLAD
QMISRIEYVHTKNFIHRDIKPDNFLMGIGRHCNKLFLIDFGLAKKYRDNRTRQHIPYRE
DKNLTGTARYASINAHLGIEQSRRDDMESLGYVLMYFNRTSLPWQGLKAATKKKKYEKI
SEKKMSTPVEVLCKGFPAEFAMYLNYCRGLRFEEAPDYMYLRQLFRILFRTLNHQYDYT
FDWTMLKQKAAQQAASSSGQGQQAQTPTGKQTDKTKSNMKGF

In another particular embodiment, the amino acid sequence of the protein encoded by the ABCC3 gene comprises the sequence SEQ ID NO: 14.
SEQ ID NO: 14

MDALCGSGELGSKFWDSNLSVHTENPDLTPCFQNSLLAWVPCIYLWVALPCYLLYLRHH
CRGYIILSHLSKLKMVLGVLLWCVSWADLFYSFHGLVHGRAPAPVFFVTPLVVGVTMLL
ATLLIQYERLQGVQSSGVLIIFWFLCVVCAIVPFRSKILLAKAEGEISDPFRFTTFYIH
FALVLSALILACFREKPPFFSAKNVDPNPYPETSAGFLSRLFFWWFTKMAIYGYRHPLE
EKDLWSLKEEDRSQMVVQQLLEAWRKQEKQTARHKASAAPGKNASGEDEVLLGARPRPR
KPSFLKALLATFGSSFLISACFKLIQDLLSFINPQLLSILIRFISNPMAPSWWGFLVAG
LMFLCSMMQSLILQHYYHYIFVTGVKFRTGIMGVIYRKALVITNSVKRASTVGEIVNLM
SVDAQRFMDLAPFLNLLWSAPLQIILAIYFLWQNLGPSVLAGVAFMVLLIPLNGAVAVK
MRAFQVKQMKLKDSRIKLMSEILNGIKVLKLYAWEPSFLKQVEGIRQGELQLLRTAAYL
HTTTTFTWMCSPFLVTLITLWVYVYVDPNNVLDAEKAFVSVSLFNILRLPLNMLPQLIS
NLTQASVSLKRIQQFLSQEELDPQSVERKTISPGYAITIHSGTFTWAQDLPPTLHSLDI
QVPKGALVAVVGPVGCGKSSLVSALLGEMEKLEGKVHMKGSVAYVPQQAWIQNCTLQEN
VLFGKALNPKRYQQTLEACALLADLEMLPGGDQTEIGEKGINLSGGQRQRVSLARAVYS
DADIFLLDDPLSAVDSHVAKHIFDHVIGPEGVLAGKTRVLVTHGISFLPQTDFIIVLAD
GQVSEMGPYPALLQRNGSFANFLCNYAPDEDQGHLEDSWTALEGAEDKEALLIEDTLSN
HTDLTDNDPVTYVVQKQFMRQLSALSSDGEGQGRPVPRRHLGPSEKVQVTEAKADGALT
QEEKAAIGTVELSVFWDYAKAVGLCTTLAICLLYVGQSAAAIGANVWLSAWTNDAMADS
RQNNTSLRLGVYAALGILQGFLVMLAAMAMAAGGIQAARVLHQALLHNKIRSPQSFFDT
TPSGRILNCFSKDIYVVDEVLAPVILMLLNSFFNAISTLVVIMASTPLFTVVILPLAVL
YTLVQRFYAATSRQLKRLESVSRSPIYSHFSETVTGASVIRAYNRSRDFEIISDTKVDA
NQRSCYPYIISNRWLSIGVEFVGNCVVLFAALFAVIGRSSLNPGLVGLSVSYSLQVTFA
LNWMIRMMSDLESNIVAVERVKEYSKTETEAPWVVEGSRPPEGWPPRGEVEFRNYSVRY

RPGLDLVLRDLSLHVHGGEKVGIVGRTGAGKSSMTLCLFRILEAAKGEIRIDGLNVADI
GLHDLRSQLTIIPQDPILFSGTLRMNLDPFGSYSEEDIWWALELSHLHTFVSSQPAGLD
FQCSEGGENLSVGQRQLVCLARALLRKSRILVLDEATAAIDLETDNLIQATIRTQFDTC
TVLTIAHRLNTIMDYTRVLVLDKGVVAEFDSPANLIAARGIFYGMARDAGLA

In another particular embodiment, the amino acid sequence of the protein encoded by the Serpine1 gene comprises the sequence SEQ ID NO: 15.
SEQ ID NO: 15

MQMSPALTCLVLGLALVFGEGSAVHHPPSYVAHLASDFGVRVFQQVAQASKDRNVVFSP
YGVASVLAMLQLTTGGETQQQIQAAMGFKIDDKGMAPALRHLYKELMGPWNKDEISTTD
AIFVQRDLKLVQGFMPHFFRLFRSTVKQVDFSEVERARFIINDWVKTHTKGMISNLLGK
GAVDQLTRLVLVNALYFNGQWKTPFPDSSTHRRLFHKSDGSTVSVPMMAQTNKFNYTEF
TTPDGHYYDILELPYHGDTLSMFIAAPYEKEVPLSALTNILSAQLISHWKGNMTRLPRL
LVLPKFSLETEVDLRKPLENLGMTDMFRQFQADFTSLSDQEPLHVAQALQKVKIEVNES
GTVASSSTAVIVSARMAPEEIIMDRPFLFVVRHNPTGTVLFMGQVIEP

In another particular embodiment, the amino acid sequence of the protein encoded by the HES1 gene comprises the sequence SEQ ID NO: 16.
SEQ ID NO: 16

Protein levels can be detected by immunohistochemistry, immunofluorescence, ELISA, western blot, preoteomic analyses, FACs, HPLC, antibody-bound magnetic beads, immunoprecipitation.

The term "functionally equivalent variant" refers to a protein (or polypeptide) comprising one or more alterations, such as substitutions, insertions, deletions and/or truncations of one or more specific amino acid residues at one or more specific positions in the protein, and having the same function that the original protein.

Thus, variants of the proteins encoded by the biomarkers of the invention may be (i) one in which one or more of the amino acid residues are substituted with a conserved or non-conserved amino acid residue (preferably a conserved amino acid residue) and such substituted amino acid residue may or may not be one encoded by the genetic code, (ii) one in which there are one or more modified amino acid residues, e.g., residues that are modified by the attachment of substituent groups, (iii) one in which the protein is an alternative splice variant of the proteins of the present invention and/or (iv) fragments of the proteins. The fragments include proteins generated via proteolytic cleavage (including multi-site proteolysis) of an original sequence. Variants are deemed to be within the scope of those skilled in the art from the teaching herein.

The term "fragment" means a protein or a domain thereof having one or more (e.g., several) amino acids absent from the amino and/or carboxyl terminus of the amino acid sequence, wherein the fragment has the same activity than the whole protein.

Practically any conventional method can be used to quantify the levels of proteins encoded by the biomarkers of the present invention. By way of non-limiting example, the levels of said proteins can be quantified by means of conventional methods, for example, using antibodies with a capacity to specifically bind to proteins (or to fragments thereof containing antigenic determinants) and subsequent quantification of the resulting antibody-antigen complexes. An example of an antibody which can be used for quantifying the levels of NAMPT protein includes, without limited to, Anti-Visfatin antibody BETHYL A300-779A.

The antibodies to be employed in these assays can be, for example, polyclonal sera, hybridoma supernatants or monoclonal antibodies, antibody fragments, Fv, Fab, Fab' y F(ab')2, ScFv, diabodies, triabodies, tetrabodies and humanised antibodies. At the same time, the antibodies can be labeled or not. Illustrative, but non-exclusive examples of markers which can be used include radioactive isotopes, enzymes, fluorophores, chemiluminescent reagents, enzymatic substrates or cofactors, enzymatic inhibitors, particles, colorants, etc. There are a wide variety of well-known assays that can be used in the present invention, which use non-labeled antibodies (primary antibody) and labeled antibodies (secondary antibodies); among these techniques are included Western-blot or Western transfer, ELISA (enzyme linked immunosorbent assay), RIA (radioimmunoassay), competitive EIA (enzymatic immunoassay), DAS-ELISA (double antibody sandwich ELISA), immunocytochemical and immunohistochemical techniques, techniques based on the use of biochips or protein microarrays including specific antibodies or assays based on colloidal precipitation in formats such as dipsticks. Other ways of detecting and quantifying protein levels include techniques of affinity chromatography, binding-ligand assays, etc.

In a particular embodiment, the levels of protein are quantified by means of western blot, ELISA, immunohistochemistry or a protein array.

As used herein, the term "subject" or refers to all the animals classified as mammals and includes, but is not restricted to, domestic and farm animals, primates and humans, for example, human beings, non-human primates, cows, horses, pigs, sheep, goats, dogs, cats, or rodents. The subject is preferably a male or female human of any age or race.

Once the expression levels of the biomarkers of the invention have been determined, the method of the invention comprises a second step [step (b)] comprising comparing the expression levels obtained in step (a) with a reference value. If the expression levels of said genes are increased with respect to the reference value, then said subject suffers from glioma, said subject has a poor prognosis, or said subject is not responding to the therapy. Examples of therapy which can be administered in order to treat glioma have been explained previous paragraphs.

The present invention the reference value or values may be based on tissue (e.g. brain tissue) obtained from, by way of example:
(a) histologically normal tissue (same or different tissue) of the subject,
(b) a similar or identical region of the brain of a second individual, or a population of individuals, of known glioma status (e.g. normal, afflicted, etc.), and
(c) a reference cell line.

In a particular embodiment, the reference value is the expression levels of each of the biomarkers of the invention in a non-tumoral cell or non-tumoral cell population obtained from the subject who is being diagnosed. This population will be compared to biopsy from the subject in study.

In another particular embodiment, the reference value is obtained from a cohort of reference patients afflicted by glioma.

By "reference patients" as it is defined in the invention is meant patients for which data regarding their survival, the evolution of their pathology, the treatment or surgery that they have received over many months or years are known.

The determination of the level of expression of the biomarkers of the invention needs to be correlated with reference values which, as mentioned above, correspond to the median value of expression levels of said biomarkers measured in a collection of tumor tissue in biopsy samples from glioma subjects. Once this median value is established, the level of this marker expressed in tumor tissues from the subject can be compared with this median value, and thus be assigned a level of "low," "normal" or "high" expression. The collection of samples from which the reference level is derived will preferably be constituted from subjects suffering from the same grade of glioma, i.e. grade I, II, III or IV.

For example, the one described in the examples which is statistically representative was constituted with data from subjects suffering from glioma coming from the French, TGCA, French-Core Exon, Sun Brain and Freije dataset which are available through Oncomine (Rhodes DR, Yu J, Shanker K, et al. ONCOMINE: A Cancer Microarray Database and Integrated Data-Mining Platform. Neoplasia (New York, NY). 2004;6(1):1-6.).

The reference value used for determining whether the expression of a gene sample is "high" (increase) or "low" corresponds to the median value of expression levels of NAMPT gene, CD44 gene, Jun gene, TEAD4 gene, CSNK1A1 gene, ABCC3 gene, Serpine1 gene and HES1 gene, measured in a RNA sample obtained by pooling equal amounts of RNA from each of the samples obtained from subjects suffering from glioma. Once this median value is established, the level of this marker expressed in the sample from the subject can be compared with this median value, and thus be assigned a level of "high" (increase) or "low" (decreased) expression.

In a particular embodiment, an increase in expression above the reference value of at least 1.1-fold, 1.5-fold, 2-fold, 5-fold, 10-fold, 20-fold, 30-fold, 40-fold, 50-fold, 60-fold, 70-fold, 80-fold, 90-fold, 100-fold or even more compared with the reference value is considered as "high" (increase) expression. In a particular embodiment, a decrease in expression below the reference value of at least 0.9-fold, 0.75-fold, 0.2-fold, 0.1-fold, 0.05-fold, 0.025-fold, 0.02-fold, 0.01-fold, 0.005-fold or even less compared with the reference value is considered as "low" (decreased) expression.

Additionally, the present invention relates to a kit or device useful to put into practice the methodology herein described. The kit or device contains the necessary reagents for detecting the expression levels of the biomarkers of the invention.

Thus, in another aspect, the invention relates to a kit or device for *in vitro* diagnosing glioma in a subject or for determining the prognosis of a subject suffering from glioma, or for determining the response of a subject to a therapy against glioma, hereinafter "kit or device of the invention", comprising
- reagents for specifically determining the expression level of the NAMPT gene, and
- reagents for specifically determining the expression level of, at least, two, three, four, five, six or seven genes selected from the group consisting of CD44, Jun, TEAD4, CSNK1A1, ABCC3, Serpine1 and HES1,
wherein said reagents are selected from the group consisting of probes, primers and/or antibodies specific for each of said genes or the corresponding proteins.

In the present invention, a "kit" or "device" is understood as a product comprising the different reagents for the detection of expression levels of gene NAMPT, and for the detection of the expression level of, at least, two, three, four, five, six or seven genes selected from the group consisting of CD44, Jun, TEAD4, CSNK1A1, ABCC3, Serpine1 and HES1.

Another component which may be present in the kit or device is a packing which allows maintaining the reagents within determined limits. Suitable materials for preparing such packings include glass, plastic (polyethylene, polypropylene, polycarbonate and the like), bottles, vials, paper, sachets and the like. The kit or device of the invention can additionally contain instructions for using the reagents in the method of the invention. Said instructions can be found in the form of printed material or in the form of an electronic support which can store instructions such that they can be read by a subject, such as electronic storage media (magnetic disks, tapes and the like), optical media (CD-ROM, DVD) and the like. The media can additionally or alternatively contain Internet websites providing said instructions.

In a particular embodiment, the reagents of the kit or device are capable of specifically detecting the levels of the mRNA encoded by the biomarkers of the invention. Reagents capable of specifically detecting the levels of the mRNA encoded by the biomarkers of the invention are:
(i) oligonucleotide primers capable of specifically amplifying a fragment of the nucleotide sequence of the NAMPT, CD44, Jun, TEAD4, CSNK1A1, ABCC3, Serpine1 and/or HES1 genes; and/or
(j) oligonucleotide probes complementary to a fragment of the nucleotide sequence of the NAMPT, CD44, Jun, TEAD4, CSNK1A1, ABCC3, Serpine1 and/or HES1 genes.

Oligonucleotide probes and primers can be generated by any suitable method known in the art (see e.g., Sambrook et al., Molecular Cloning: A Laboratory Manual, 4thEd., Cold Spring Harbor Press, Cold Spring Harbor, N.Y., 2012). For example, polynucleotide probes that specifically bind to the mRNA transcripts of the genes described herein (or cDNA synthesized therefrom) can be created using the nucleic acid sequences of the mRNA or cDNA targets themselves by routine techniques (e.g., PCR or synthesis). As used herein, the term "fragment" means a contiguous part or portion of a polynucleotide sequence comprising about 10 or more nucleotides, about 15 or more nucleotides, about 20 or more nucleotides, about 30 or more, or even about 50 or more nucleotides. The exact nature of the polynucleotide probe is not critical to the invention; any probe that will selectively bind the mRNA or cDNA target can be used. In a particular embodiment, the oligonucleotide primers and probes of the kit or device of the invention have a size of between 10 and 150 nucleotides and hybridize under low, medium or high stringency conditions with the nucleotide sequence of the gen which is going to be amplified or detected.

As defined in the method of the invention, the nucleotide sequences of the genes which are going to be amplified or detected are the following:
The NAMPT gene comprises a nucleotide sequence comprising a sequence identity of, at least, 50, 55, 60, 65, 70, 75, 80, 85, 90, 95, 96, 97, 98 or 99 % with the nucleotide sequence SEQ ID NO: 1. Nevertheless, in a more particular embodiment, the NAMPT gene comprises the sequence SEQ ID NO: 1.

The CD44 gene comprises a nucleotide sequence comprising a sequence identity of, at least, 50, 60, 65, 70, 75, 80, 85, 90, 95, 96, 97, 98 or 99 % with the nucleotide sequence SEQ ID NO: 2. Nevertheless, in a more particular embodiment, the CD44 gene comprises the sequence SEQ ID NO: 2.

The Jun gene comprises a nucleotide sequence comprising a sequence identity of, at least, 50, 60, 65, 70, 75, 80, 85, 90, 95, 96, 97, 98 or 99 % with the nucleotide sequence SEQ ID NO: 3. Nevertheless, in a more particular embodiment, the Jun gene comprises the sequence SEQ ID NO: 3.

The TEAD4 gene comprises a nucleotide sequence comprising a sequence identity of, at least, 50, 60, 65, 70, 75, 80, 85, 90, 95, 96, 97, 98 or 99 % with the nucleotide sequence SEQ ID NO: 4. Nevertheless, in a more particular embodiment, the TEAD4 gene comprises the sequence SEQ ID NO: 4.

The CSNK1A1 gene comprises a nucleotide sequence comprising a sequence identity of, at least, 50, 60, 65, 70, 75, 80, 85, 90, 95, 96, 97, 98 or 99 % with the nucleotide sequence SEQ ID NO: 5. Nevertheless, in a more particular embodiment, the CSNK1A1 gene comprises the sequence SEQ ID NO: 5.

The ABCC3 gene comprises a nucleotide sequence comprising a sequence identity of, at least, 50, 60, 65, 70, 75, 80, 85, 90, 95, 96, 97, 98 or 99 % with the nucleotide sequence SEQ ID NO: 6. Nevertheless, in a more particular embodiment, the ABCC3 gene comprises the sequence SEQ ID NO: 6.

The Serpine1 gene comprises a nucleotide sequence comprising a sequence identity of, at least, 50, 60, 65, 70, 75, 80, 85, 90, 95, 96, 97, 98 or 99 % with the nucleotide sequence SEQ ID NO: 7. Nevertheless, in a more particular embodiment, the Serpine1 gene comprises the sequence SEQ ID NO: 7.

The HES1 gene the HES1 gene comprises a nucleotide sequence comprising a sequence identity of, at least, 50, 60, 65, 70, 75, 80, 85, 90, 95, 96, 97, 98 or 99 % with the nucleotide sequence SEQ ID NO: 8. Nevertheless, in a more particular embodiment, the HES1 gene comprises the sequence SEQ ID NO: 8.

In the context of the present invention, the expression "stringent conditions" refers to the strictness with which, during nucleic acid hybridization, the Watson-Crick base-pairing is required under specified conditions of temperature, pH, salt concentration, etc. Conditions of high stringency require all bases of one polynucleotide to be paired with complementary bases on the other; conditions of medium stringency allow some bases to be unpaired. Medium or high stringency conditions between any two nucleotide sequences are widely known in the state of art depending on the number and type of bases of the nucleotide sequence.

"High stringency conditions," as defined herein, are identified by, but not limited to, those that: (1) use low ionic strength and high temperature for washing, for example 0.015 M sodium chloride/0.0015 M sodium citrate/0.1% sodium dodecyl sulfate at 50° C.; (2) use during hybridization a denaturing agent, such as formamide, for example, 50% (v/v) formamide with 0.1% bovine serum albumin/0.1% Ficoll/0.1% polyvinylpyrrolidone/50 mM sodium phosphate buffer at pH 6.5 with 750 mM sodium chloride, 75 mM sodium citrate at 42° C.; or (3) use 50% formamide, 5×SSC (0.75 M NaCl, 0.075 M sodium citrate), 50 mM sodium phosphate (pH 6.8), 0.1% sodium pyrophosphate, 5×Denhardt's solution, sonicated salmon sperm DNA (50 µg/ml), 0.1% SDS, and 10% dextran sulfate at 42° C., with washes at 42° C. in 0.2×SSC (sodium chloride/sodium, citrate) and 50% formamide at 55° C., followed by a high-stringency wash consisting of 0.1×SSC containing EDTA at 55° C.

"Medium stringent conditions" are described by, but not limited to, those in Sambrook et al., Molecular Cloning: A Laboratory Manual, New York: Cold Spring Harbor Press, 1989, and include the use of washing solution and hybridization conditions (e.g., temperature, ionic strength and % SDS) less stringent than those described above. An example of moderately stringent conditions is overnight incubation at 37° C. in a solution comprising: 20% formamide, 5×SSC (150 mM NaCl, 15 mM trisodium citrate), 50 mM sodium phosphate (pH 7.6), 5×Denhardt's solution, 10% dextran sulfate, and 20 mg/mL denatured sheared salmon sperm DNA, followed by washing the filters in 1×SSC at about 37-50° C. The skilled artisan will recognize how to adjust the temperature, ionic strength, etc. as necessary to accommodate factors such as probe length and the like.

As the skilled person understands, the oligonucleotides of the kit or device of the invention can be use in all techniques of gene expression profiling (RT-PCR, SAGE, TaqMan, Real Time- PCR, etc.).

In another embodiment, the reagents of the kit or device are capable of specifically detecting the levels of the proteins encoded by the biomarkers of the invention. Reagents capable of specifically detecting the expression levels of the proteins encoded by the NAMPT, CD44, Jun, TEAD4, CSNK1A1, ABCC3, Serpine1 and/or HES1 genes, are antibodies with a capacity to specifically bind to said proteins (or to fragments thereof containing antigenic determinants). Examples of the antibodies to be employed in the present invention have been previously cited. The antibodies of the kit of the invention can be used in conventional methods for detecting protein expression levels, such as Western-blot or Western transfer, ELISA (enzyme linked immunosorbent assay), RIA (radioimmunoassay), competitive EIA (enzymatic immunoassay), DAS-ELISA (double antibody sandwich ELISA), immunocytochemical and immunohistochemical techniques, techniques based on the use of biochips, protein microarrays including specific antibodies or assays based on colloidal precipitation in formats such as dipsticks, etc.

The terms "kit" or "device" may also encompass arrays. As the skilled person known, a convenient way of measuring RNA transcript levels for multiple genes in parallel is to use an array (also referred to as microarrays in the art). Techniques for using arrays to assess and compare gene expression levels are well known in the art and include appropriate hybridization, detection and data processing protocols. A useful array includes multiple polynucleotide probes (typically DNA) that are immobilized on a solid substrate (e.g. a glass support such as a microscope slide, or a membrane) in separate locations (e.g., addressable elements) such that detectable hybridization can occur between the probes and the transcripts to indicate the amount of each transcript that is present. The arrays disclosed in this application can be used in methods of detecting the expression of a desired combination of genes, which combinations are discussed throughout this application.

As used herein, the term "addressable element" means an element that is attached to the substrate at a predetermined position and specifically binds a known target molecule, such that when target-binding is detected (e.g., by fluorescent labeling), information regarding the identity of the bound molecule is provided on the basis of the location of the element on the substrate. Addressable elements are "different" for the purposes of the present disclosure if they do not bind to the same target gene. The addressable element comprises one or more polynucleotide probes specific for an snRNA transcript of a given gene, or a cDNA synthesized from the mRNA transcript. The addressable element can comprise more than one copy of a polynucleotide, can comprise more than one different polynucleotide, provided that all of the polynucleotides bind the same target molecule. Where a gene is known to express more than one mRNA transcript, the addressable element for the gene can comprise different probes for different transcripts, or probes designed to detect a nucleic acid sequence common to two or more (or all) of the transcripts. Alternatively, the array can comprise an addressable element for the different transcripts. The addressable element also can comprise a detectable label, suitable examples of which are well known in the art.

The substrate can be any rigid or semi-rigid support to which polynucleotides can be covalently or non-covalently attached. Suitable substrates include membranes, filters, chips, slides, wafers, fibers, beads, gels, capillaries, plates, polymers, microparticles, and the like. Materials that are suitable for substrates include, for example, nylon, glass, ceramic, plastic, silica, aluminosilicates, borosilicates, metal oxides such as alumina and nickel oxide, various clays, nitrocellulose, and the like.

The polynucleotides of the addressable elements (also referred to as "probes") can be attached to the substrate in a pre-determined 1- or 2-dimensional arrangement, such that the pattern of hybridization or binding to a probe is easily correlated with the expression of a particular gene. Because the probes are located at specified locations on the substrate (i.e., the elements are "addressable"), the hybridization or binding patterns and intensities create a unique expression profile, which can be interpreted in terms of expression levels of particular genes and can be correlated with glioma in accordance with the methods described herein.

The array can comprise other elements common to polynucleotide arrays. For instance, the array also can include one or more elements that serve as a control, standard, or reference molecule, such as a housekeeping gene or portion thereof (e.g., PBGD or GAPDH), to assist in the normalization of expression levels or the determination of nucleic acid quality and binding characteristics, reagent quality and effectiveness, hybridization success, analysis thresholds and success, etc. These other common aspects of the arrays or the addressable elements, as well as methods for constructing and using arrays, including generating, labeling, and attaching suitable probes to the substrate, consistent with the invention are well-known in the art. Other aspects of the array are as previously described herein with respect to the methods of the invention.

An array can also be used to measure protein levels of multiple proteins in parallel. Such an array comprises one or more supports bearing a plurality of ligands that specifically bind to a plurality of proteins. The ligands are optionally attached to a planar support or beads. In one embodiment, the ligands are antibodies. The proteins that are to be detected using the array correspond to the proteins encoded by the nucleic acids of interest, as described above, including the specific gene expression profiles disclosed. Thus, each ligand (e.g. antibody) is designed to bind to one of the target proteins. As with the nucleic acid arrays, each ligand is preferably associated with a different addressable element to facilitate detection of the different proteins in a sample.

The different uses of the kit or device of the invention constitute additional aspects of the present invention.

Thus, in another aspect, the invention relates to the use *in vitro* of the kit or device of the invention for diagnosing glioma in a subject, for determining the prognosis of a subject suffering from glioma, or determining the response of a subject to a therapy against glioma. As explained previously, if said agents detect an increase expression levels of the gene NAMPT with respect to a reference levels and, an increase expression levels of, at least, two, three, four, five, six or seven genes selected from the group consisting of CD44, Jun, TEAD4, CSNK1A1, ABCC3, Serpine1 and HES1 with respect to said reference levels, then said subject suffers from glioma, said subject has a poor prognosis, or said subject is not responding to the therapy. The reference levels, as well as the meaning the terms used in the present aspect, have been defined previously in the present description.

In another aspect, the present invention relates to the use *in vitro* of the expression levels of NAMPT gene and, additionally, the expression levels of, at least, two, three, four, five, six or seven genes from the list consisting of CD44, Jun, TEAD4, CSNK1A1, ABCC3, Serpine1 and HES1 for diagnosing glioma in a subject, for determining the prognosis of a subject suffering from glioma, or determining the response of a subject to a therapy against glioma.

In another aspect, the present invention relates to a product selected from the list consisting of a NAMPT inhibitor, an angiogenesis inhibitor, preferably an VEGF inhibitor, Opdivo, Rintega, Temozolomide (TMZ), ICT-107, Avastin, ABT-414 in combination with TMZ, ITK-1, Toca511 in combination with Toca-FC, Avastin in combination with VB-111, Veliparib in combination with TMZ, DCVax-L in combination with TMZ, and combinations thereof, for use in the treatment of a subject who has been diagnosed by a method of the invention as suffering from glioma, wherein the reference value for each gene is the expression levels of each gene in a non-tumoral cell or non-tumoral cell population obtained from the subject.

In a particular embodiment, the NAMPT inhibitor is selected from the group consisting of GNE-617, OT-82, MPC-8640, MPC-9528 and combinations thereof.

In the context of the present invention, the term "treating" (or "treat" or "treatment") refers to processes involving a slowing, interrupting, arresting, controlling, stopping, reducing, or reversing the progression or severity of an existing symptom, disorder, condition, or disease, but does not necessarily involve a total elimination of all disease-related symptoms, conditions, or disorders associated with glioma cancer

The treatment of a disorder or disease may, for example, lead to a halt in the progression of the disorder or disease (e.g., no deterioration of symptoms) or a delay in the progression of the disorder or disease (in case the halt in progression is of a transient nature only). The "treatment" of a disorder or disease may also lead to a partial response (e.g., amelioration of symptoms) or complete response (e.g., disappearance of symptoms) of the subject/patient suffering from the disorder or disease. Accordingly, the "treatment" of a disorder or disease may also refer to an amelioration of the disorder or disease, which may, e.g., lead to a halt in the progression of the disorder or disease or a delay in the progression of the disorder or disease. Such a partial or complete response may be followed by a relapse. It is to be understood that a subject/patient may experience a broad range of responses to a treatment (such as the exemplary responses as described herein above). The treatment of glioma may, *inter alia,* comprise curative treatment (preferably leading to a complete response and eventually to healing of the disorder or disease) and palliative treatment (including symptomatic relief).

In another aspect, the present invention relates to a computer program comprising program instructions for causing a computer, or the device of the invention to carry out the method of the invention.

In accordance with all aspects and embodiments of the invention, the methods provided may be computer-implemented.

Gene expression levels can be analyzed and associated with status of a subject (e.g., presence of glioma or prognosis in a digital computer. Optionally, such a computer is directly linked to a scanner or the like receiving experimentally determined signals related to gene expression levels. Alternatively, expression levels can be input by other means. The computer can be programmed to convert raw signals into expression levels (absolute or relative), compare measured expression levels with one or more reference expression levels, or a scale of such values. The computer can also be programmed to assign values or other designations to expression levels based on the comparison with one or more reference expression levels, and to aggregate such values or designations for multiple genes in an expression profile. The computer can also be programmed to output a value or other designation providing an indication of the presence of glioma or prognosis of a subject suffering from glioma as well as any of the raw or intermediate data used in determining such a value or designation.

A typical computer includes a bus which interconnects major subsystems such as a central processor, a system memory, an input/output controller, an external device such as a printer via a parallel port, a display screen via a display adapter, a serial port, a keyboard, a fixed disk drive and a port (e.g., USB port) operative to receive an external memory storage device. Many other devices can be connected such as a scanner via I/O controller, a mouse connected to serial port or a network interface. The computer contains computer readable media holding codes to allow the computer to perform a variety of functions. These functions include controlling automated apparatus, receiving input and delivering output as described above. The automated apparatus can include a robotic arm for delivering reagents for determining expression levels, as well as small vessels, e.g., microtiter wells for performing the expression analysis.

In one embodiment, any of the computer-implemented methods of the invention may comprise a step of obtaining by at least one processor information reflecting the expression level of NAMPT gene and, additionally, at least, two, three, four, five, six or seven genes from the list consisting of CD44, Jun, TEAD4, CSNK1A1, ABCC3, Serpine1 and HES1 genes in an isolated biologial sample from the subject.

In one embodiment, the computer-implemented methods comprise obtaining, by at least one processor, information reflecting the expression level of NAMPT gene and, at least, two, three, four, five, six or seven genes from the list consisting of CD44, Jun, TEAD4, CSNK1A1, ABCC3, Serpine1 and HES1 genes.

In another embodiment of the computer-implemented methods of the invention, the methods may additionally comprise the steps of i) determining by at least one processor a difference between the expression level of NAMPT gene and, at least, two, three, four, five, six or seven genes from the list consisting of CD44, Jun, TEAD4, CSNK1A1, ABCC3, Serpine1 and HES1 genes and a reference level; and (ii) outputting in user readable format the difference obtained in the determining step.

In another embodiment of the computer-implemented methods of the invention, the methods may further comprise outputting in user readable format a determination that the subject suffers from glioma, has a poor prognosis or is not responding to the therapy.

The terms and expressions used in the present inventive aspect have been described and defined in previous paragraphs.

In another aspect, the invention relates to a method of treatment of a subject suffering from glioma, hereinafter "method of treatment of the invention", comprising
(a) determining the expression levels of NAMPT gene and, additionally, the expression levels of, at least, two, three, four, five, six or seven genes from the list consisting of CD44, Jun, TEAD4, CSNK1A1, ABCC3, Serpine1 and HES1 in an isolated biological sample from the subject, and
(b) comparing the expression levels obtained in step (a) with a reference value, wherein if the expression levels of said genes are increased with respect to the reference value, then the subject is treated with a product selected from the list consisting of an angiogenesis inhibitor, Opdivo, Rintega, Temozolomide (TMZ), ICT-107, Avastin, ABT-414 in combination with TMZ, ITK-1, Toca511 in combination with Toca-FC, Avastin in combination with VB-111, Veliparib in combination with TMZ, DCVax-L in combination with TMZ, and combinations thereof, and
wherein the reference value for each gene is the expression levels of each gene in a non-tumoral cell obtained from the subject.

In a particular embodiment of the method of treatment, the angiogenesis inhibitor is preferably VEGF inhibitors and more preferably Bevazizumab. In another particular embodiment, the angiogenesis inhibitor is administered in combination with a NAMPT inhibitor, preferably, the NAMPT inhibitor is selected from the group consisting of GNE-617, OT-82, MPC-8640, MPC-9528, and combinations thereof.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples, drawings and sequence listing are provided by way of illustration and are not intended to be limiting of the present invention.

### BRIEF DESCRIPTION OF THE DRAWINGS

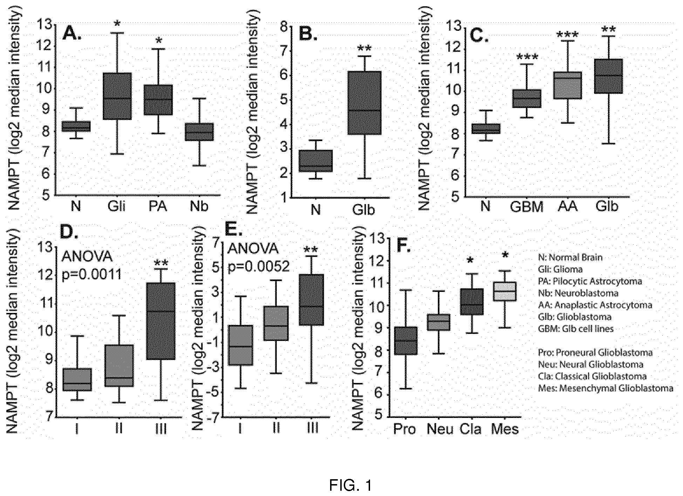

**Figure 1****. NAMPT Expression Correlates with Tumor Grade.** (A) Analysis of normal brain [n=216], glioma [n=577], pilocytic astrocytoma [n=41] and neuroblastoma [n=45] datasets available through the R2 database indicates that NAMPT is significantly upregulated in tumors derived from the ectoderm, such as pilocytic astrocytoma [*, p=0.01 with ANOVA compared to normal brain] and glioma [*, p=0.01 with ANOVA compared to normal brain]. (B) Analysis of normal brain [n=23] and glioblastoma [n=81] datasets from the Sun database available in Oncomine indicates that NAMPT is highly overexpressed in glioblastoma [**, p<0.01 with ANOVA compared to normal
   brain]. (C) New retrospective analyses of new databases comparing normal brain [n=216], glioblastoma [n=9], anaplastic astrocytoma - glioma grade III [n=16] and glioblastoma [n=159] cell lines shows that NAMPT is upregulated in the mRNA pools of these cell lines [****, p<0.0001 with ANOVA compared to normal brain] and that its expression is upregulated at baseline in glioma grade III cells [***, p<0.001 with ANOVA compared to normal brain]. (D-E) The Oncomine glioma dataset shows a correlation between NAMPT expression and tumor grade. (D) Analysis of the Oncomine glioma dataset with respect to NAMPT expression in grade II [n=45], (E) grade III [n=31] and grade IV [n=81] tumors shows a significant difference in NAMPT expression between grade II and grade IV tumors [**, p<0.001]. (F) Analysis of TCGA Glioblastoma molecular subtypes Proneural, Neural, Classical and Mesenchymal.

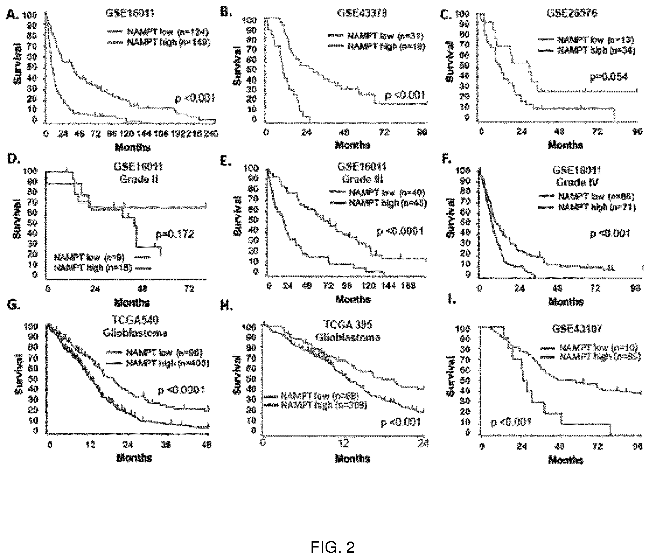

**Figure 2****. NAMPT is an independent indicator of Glioma Patient Outcomes.** Analysis of glioma datasets available through Oncomine and R2 indicates the existence of a significant correlation between high NAMPT expression and poor survival in the French tumor glioma dataset (A) [n=124 NAMPT low; n=149 NAMPT high; p=3,20E- 13 with log-rank analysis]; Kawaguchi tumor glioma dataset. (B) [n=31 NAMPT low; n=19 NAMPT high; p=1.3E-05 with logrank analysis]; Paugh tumor glioma dataset. (C) [n=13 NAMPT low; n=34 NAMPT high; p=0.054 with log-rank analysis]; French tumor glioma dataset, subtype grade II. (D) [n=9 NAMPT low; n=15 NAMPT high; p=0.172 with log-rank analysis]; French tumor glioma dataset, subtype grade III. (E) [n=40 NAMPT low; n=45 NAMPT high; p=0.000018 with log-rank analysis]; and French tumor glioma dataset, subtype grade IV. (F) [n=85 NAMPT low; n=71 NAMPT high; p=0.000360 with log-rank analysis]. The poor prognosis of grade IV glioblastoma is confirmed via analysis of the TCGA 540 glioblastoma dataset [n=96 NAMPT low; n=408 NAMPT high; p=0.000071 with log-rank analysis] (G), TCGA 395 glioblastoma dataset [n=68 NAMPT low; n=309 NAMPT high; p=0.00089 with logrank analysis] (H) and French Exon-Core dataset [n=85 NAMPT low; n=10 NAMPT high; p=0.0026 with log-rank analysis] (I).

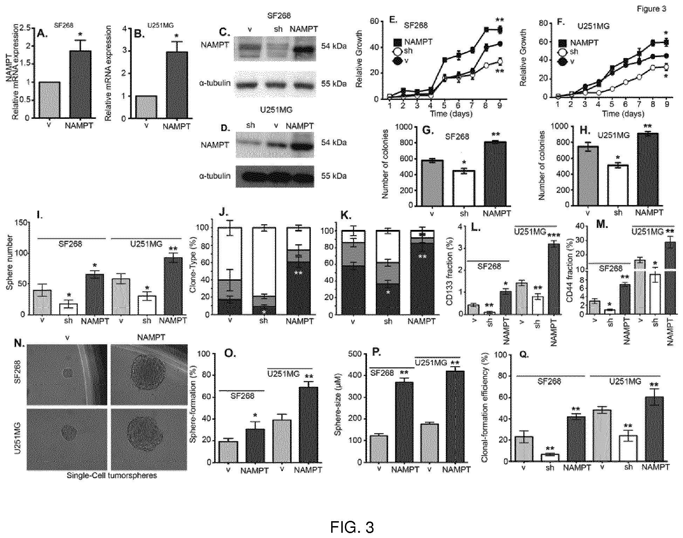

**Figure 3****. NAMPT Expression Increases Tumorigenic and CIC properties.** (A, B) qRT PCR shows NAMPT overexpression in either SF268 (A) or U251MG (B). (C, F) Western blot analysis shows NAMPT overexpression and NAMPT silencing with shRNA in SF268 (C) and U251MG (D). (E,F) Analysis of the growth curve indicates that NAMPT overexpression confers a proliferative advantage [*, p<0.05; **, p<0.01 with ANOVA compared to vector], whereas NAMPT underexpression slows proliferation [*, p<0.05, **, p<0.01 with ANOVA compared to vector]. (G, H) Clonogenicity assay results indicate that NAMPT overexpression increases the number of colonies [**, p<0.01 with ANOVA compared to vector], whereas NAMPT underexpression decreases the number of colonies [*, p<0.05 with ANOVA compared to vector]. (I) Tumorsphere-forming assay in both cell lines indicates that NAMPT overexpression increases both number and size, whereas NAMPT underexpression decreases tumorsphere number and size. (J, K) Analysis of clone phenotypes [holoclones - black, meroclones - gray, paraclones - white] shows that NAMPT overexpression increases the number of holoclones [**, p<0.01 with ANOVA compared to vector], whereas NAMPT underexpression decreases the holoclone number [*, p<0.05 with ANOVA compared to vector]. (L) CD133 analysis with FACS indicates that NAMPT overexpression increases CD133 levels [*, p<0.05 with ANOVA compared to vector], whereas NAMPT underexpression decreases CD133 levels [**, p<0.01 with ANOVA compared to vector]. (M) CD44 analysis with FACS indicates that NAMPT overexpression increases CD44 levels [**, p<0.01 with ANOVA compared to vector], whereas NAMPT underexpression decreases CD44 levels [*, p<0.05 with ANOVA compared to vector]. (N-Q) Single-cell Sphere-forming assay indicates that NAMPT overexpression increases both number and size, whereas NAMPT underexpression decreases tumorsphere number and size. (N) Single cell tumorsphere forming efficiency representative picture. (O) Single cell tumorsphere forming efficiency percentage. (Q) Single cell tumorsphere size. (Q) Single cell colony (full culture) forming efficiency percentage.

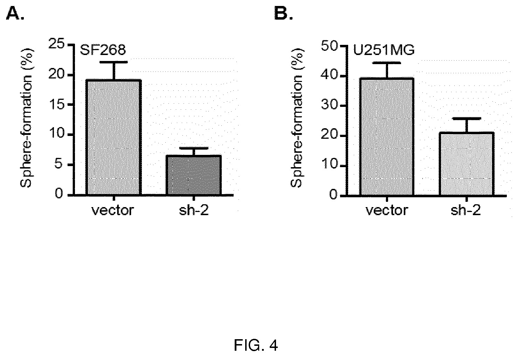

**Figure 4****. Sphere-forming efficiency associated to the causal effect of a second sh expressed on SF268 and U251 MG cell lines**. We show similar results to the sh-1 using a second sh-2 against NAMPT. We measured the sphere-forming capacity from a single cell on SF268 (A) and U251MG (B) showing similar results to the sh-1.

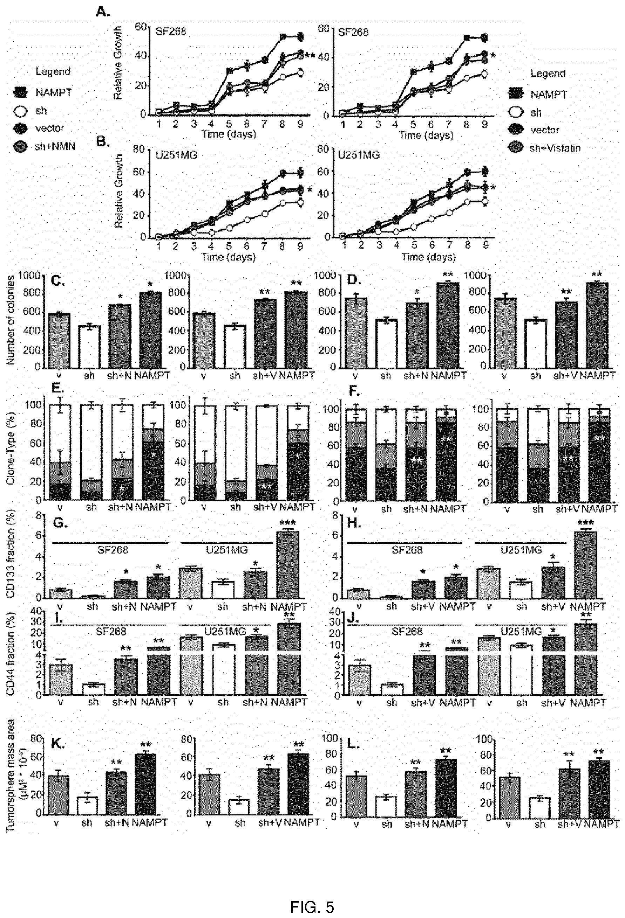

**Figure 5****. NAMPT-driven tumorigenic and CIC properties are rescued both metabolically and enzymatically**. (A) left - Analysis of the growth curve for SF268 NAMPT-underexpressing cells indicates that 1 mM NMN can rescue the vector-proliferation ratio [**, p<0.01 with ANOVA compared to sh]. right - 60 ng/mL visfatin has the same effect [*, p<0.05 with ANOVA compared to sh]. (B) Same results described for U251MG [*, p<0.05 with ANOVA compared to sh] (C) left - Analysis of clonogenicity in SF268 NAMPT-underexpressing cells indicates that 1 mM NMN can rescue the clonogenetic ability of the vector [*, p<0.05 with ANOVA compared to sh] right - 60 ng/mL visfatin has the same effect [**, p<0.01 ANOVA compared to sh]. (D) Same results described for U251MG [*, p<0.05; **, p<0.01 with ANOVA compared to sh] (E) Analysis of clone phenotypes in NAMPT- underexpressing cells indicates that 1 mM NMN [*, p<0.05; **, p<0.01 with ANOVA compared to sh] and (F) 60 ng/mL visfatin [**, p<0.01 with ANOVA compared to sh] can rescue the number of holoclones in the vector in both cell lines. (G) CD133 analysis of SF268 and U251MG NAMPT-underexpressing cells indicates that 1 mM NMN [*, p<0.05; ***, p<0.001 with ANOVA compared to sh] and (H) 60 ng/mL visfatin [*, p<0.05; ***, p<0.001 with ANOVA comparison to sh] can rescue and surpass the levels of CD133 expression in the vector of both cell lines. (I) CD44 analysis of low SF268 and U251MG NAMPT cells indicates that 1 mM NMN [*, p<0.05; **, p<0.01; ***, p<0.001 with ANOVA compared to sh] and (J) 60 ng/mL visfatin [*, p<0.05; **, p<0.01with ANOVA compared to sh] can rescue the levels of CD44 expression in the vector of both cell lines. (K) left - Analysis of tumorsphere area in SF268 NAMPT-underexpressing cells indicates that 1 mM NMN [**, p<0.01 with ANOVA compared to sh] and (K) - right 60 ng/mL visfatin [**, p<0.01 with ANOVA compared to sh] can rescue the number of tumorspheres generated in the vector. (L) Similar results described for U251MG. V: cells expressing vector only. NAMPT: Cells overexpressing ectopic NAMPT cDNA, Sh: cells expressing an specific NAM shRNA.

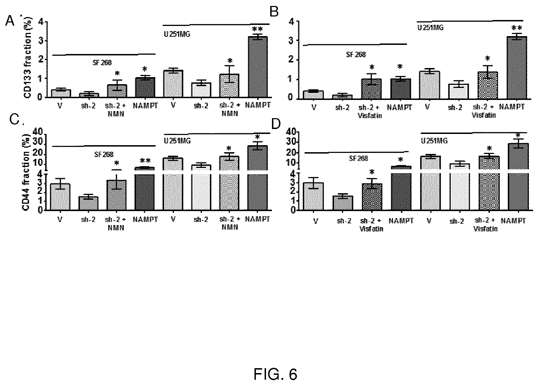

**Figure 6****. NAMPT-driven tumorigenic and CIC properties are rescued both metabolically and enzymatically, using a second shRNA.**

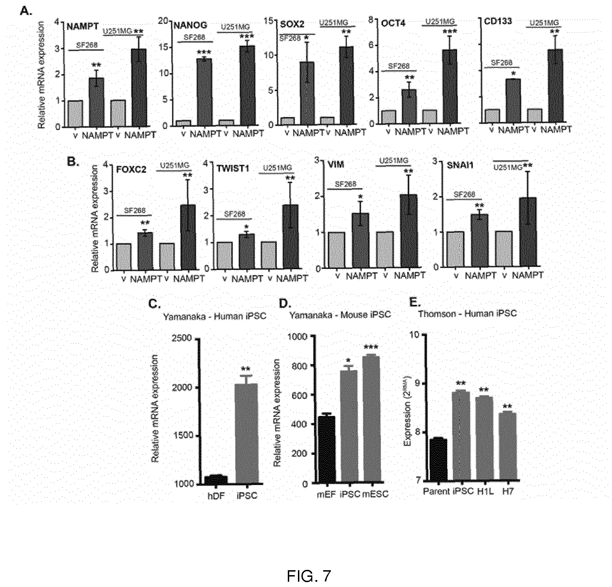

**Figure 7****. NAMPT Activates EMT and Stem Pathway Effectors.**
   (A) Q-RT-PCR analysis of NAMPT overexpression in empty vector [**, p=0.0055 with t-test]. (E) RT-qPCR analysis of stem cell gene expression in empty vector: NANOG [***, p<0.001 with t-test], SOX2 [*, p=0.0101 with t-test], OCT4 [ **, p=0.0088 with t-test] and CD133 [*, p=0.0158 with t-test]. (B) RT-qPCR analysis of EMT gene expression in empty vector: FOXC2 [**, p=0.0042 with t-test], TWIST1 [*, p=0.0132 with t-test], VIM [*, p=0.0492 with t-test] and SNAI1 [**, p=0.0023 with t-test]. (C) Analysis of the Yamanaka dataset (GSM241846) with respect to induced pluripotent stem cell (iPSC) generation from human dermal fibroblasts (hDF) demonstrates an increase in NAMPT levels with reprogramming [*, p=0.01; ** p<0.01 with ANOVA compared to human dermal fibroblasts]. (D) Analysis of the Yamanaka dataset (GSE15148) with respect to iPSC generation from mouse embryonic fibroblasts (mEF) demonstrates a significant increase in NAMPT expression with reprogramming. Mouse embryonic stem cells (mESC) also express high levels of NAMPT compared to fibroblasts [*, p=0.01; ** p<0.01 with ANOVA compared to mouse embryonic fibroblasts]. (E) Analysis of the Thomson dataset (GSE5259) with respect to iPSC generation from human foreskin fibroblasts (parent) demonstrates an increase in NAMPT levels with reprogramming. Human embryonic stem cell lines (H1L and H7) also express high levels of NAMPT compared to fibroblasts [*, p=0.01; ** p<0.01 with ANOVA compared to human foreskin fibroblasts]. V: cells expressing vector only. NAMPT: Cells overexpressing ectopic NAMPT cDNA.

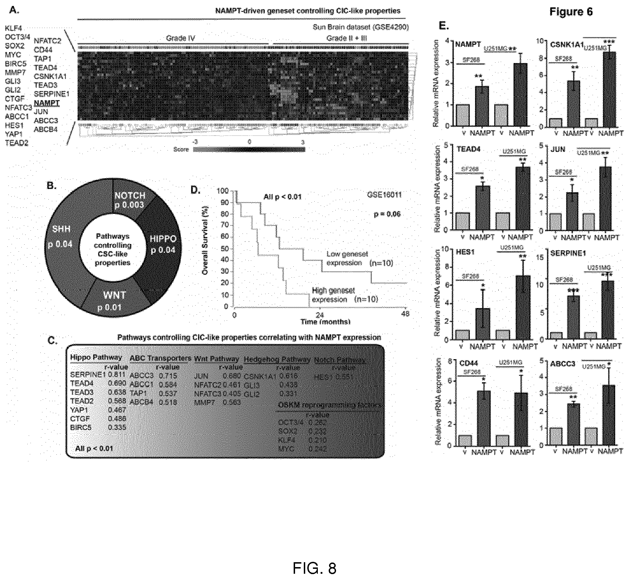

**Figure 8****. NAMPT induces a Gene Signature that Correlates with Glioma Tumor Grade and Predicts Poor Survival**. (A) R2 expression analysis of the gene signature triggered by NAMPT in the Sun dataset shows a correlation between NAMPT expression and advanced glioma stage: grade IV. R2 expression analysis of the gene signature triggered by NAMPT showed genes related to Stem Cell phenotype. (B) pathways controlling CIC-like propersies related to NAMPT expression. (C) Genes associated to NAMP gene expression [all p<0.01, Pearson r is shown in each case], and the pathway each gene is associated to. (D) Overall survival probability comparing the patients showing low expression signature vs high expression signature. R2 expression analysis of the gene signature triggered by NAMPT in the Sun Brain tumor database in a biased cohort of patients with high signature expression [n=10] and low signature expression [n=10] shows poor survival in patients [p=0.06 with log-rank analysis]. (E) Analisis of the expression of the different genes of the signature in SF268 and U2521 MG expressing NAMPT.

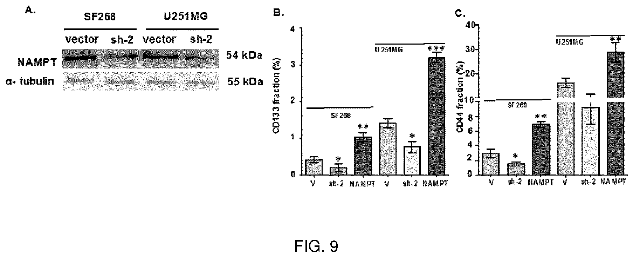

**Figure 9****. CICs related features associated to the causal effect of a second sh expressed on SF268 and U251 MG cell lines**. We show similar results to the sh-1 using a sh-2 (A) in CICs related properties such CD44 and CD133 levels.

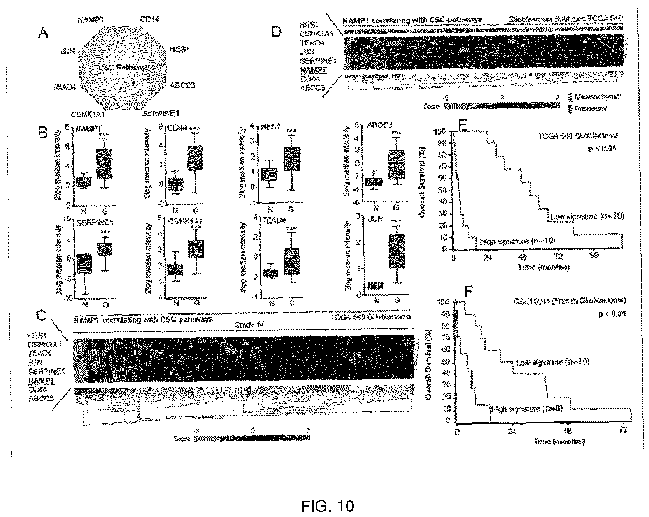

**Figure 10****. Analysis of NAMPT-related signature in the TCGA database**. (A) Nampt-derived signature is represented. (B). Relative expression leves of each gene are represented in normal brain vs glioma samples. (C) Nampt-derived signature levels clasiffy Grade IV gliobastoma samples. (D) Nampt-derived signature levels classify the different subtypes of Glioblastoma according to the whole dataset of Glioblastoma TCGA dataset. (E-F) Overall survival probability comparing the patients showing low expression signature vs high expression signature. R2 expression analysis of the gene signature triggered by NAMPT in the TCGA 540 (E) or French (F) glioblastomma databases in a biased cohort of patients with high signature expression [n=10] and low signature expression [n=10] shows poor survival in patients [p<0.01 with log-rank analysis].

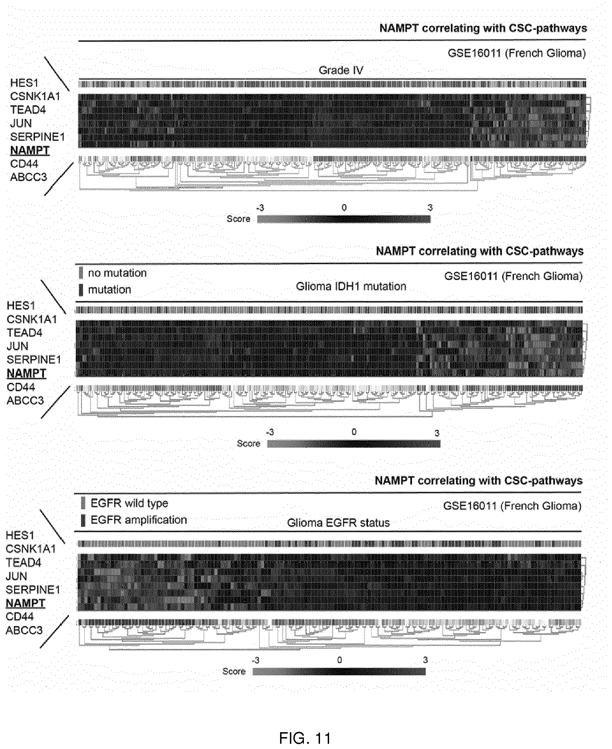

**Figure 11****. NAMPT induces a Gene Signature that Correlates with WT IDH1 patients and EGFR positive tumors.** To this study we have selected the French database that contains information about the IDH1 and EGFR mutational status. (A) Nampt-derived signature is present in a high percentage of Grade IV gliobastoma samples from French glioma database. (B). Nampt-derived signature predicts an enrichment of IDH1 mutatins in patients negative for our signature, indicating a better prognosis in patients with IDH1 mutations. (C) On the other hand, most, if not all, gliomas with EGFR amplifications strong positive correlation with our NAMPT-derived signature.

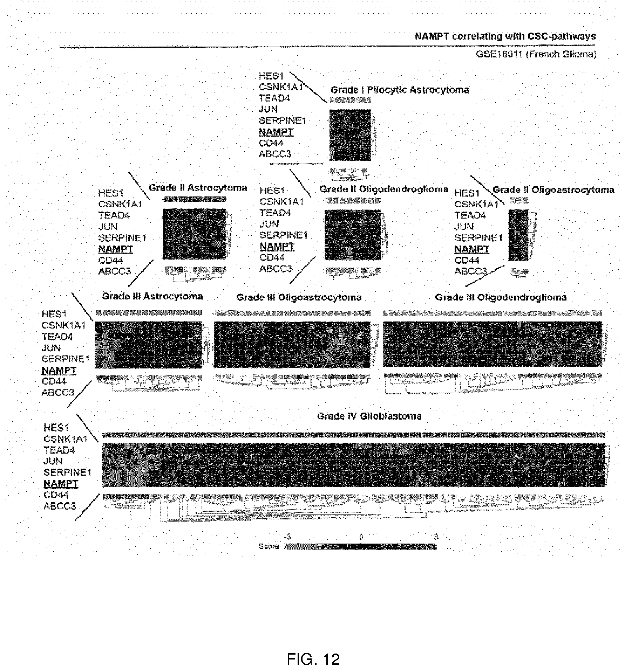

**Figure 12****. NAMPT correlating with CSC-pathways.**

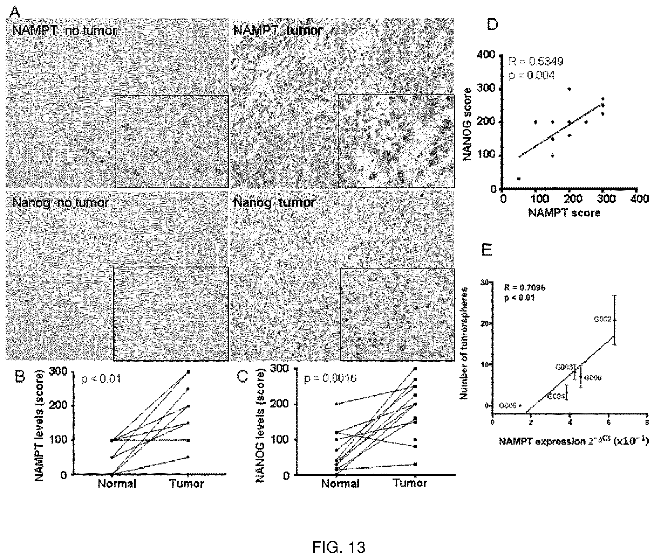

**Figure 13****. NAMPT expression correlated with high levels of cancer stem cell-like cells in glioblastoma extracted directly from patients**. A) We first took 14 **glioblastoma** tumor samples directly from patients as well as matched non-tumor samples and generated a tissue microarray, TMA. These TMAs were stained for NAMPT and Nanog according to M&M. In these samples we evaluated the expression of NAMPT and Nanog, as surrogated marker of cancer stem cell-like levels of these tumors. B) Levels of NAMPT and C) Nanog were related in matched samples, from the same patient. D) Evaluation of the correlation between matched samples of NAMPT and Nanog. There was a clear correlation (pearson r=0.53, p<0.01) between the expression of Nanog and NAMPT. E) We took 5 fresh glioblastoma samples from patients. After tissue disaggregation, we directly measured NAMPT levels by Q-RT-PCR and parallely seeded 3000 cells to measure the number of tumorspheres formed. Then we plotted to establish 1 to 1 correlation, between the level of NAMPT and the number of tumorspheres. We found a strong direct correlation of the tumorsphere number and NAMPT levels for each tumor.

### EXAMPLES

### Example 1

### I. MATERIAL AND METHODS

### Cell culture and cell transfection

The human Glioblastoma cell line SF268 and U251MG were cultured in RPMI 1640 medium (SIGMA) supplemented with 10% Fetal Bovine Serum (FBS) at 37°C under 5% CO2 atmosphere. For DNA transfection, SF268 and U251MG were transfected with Mirus TransIT-X2 Dynamic Delivery System (Mirus MIR6000) in exponential phase with 2.5 µg of a small hairping RNA (shRNA) against NAMPT (QIAGEN SureSilencing shRNA insert sequence: 5'-AAGATCCTGTTCCAGGCTATT-3' (SEQ ID NO: 17)), 2.5 µg of DNA carrying an hygromycin empty vector (QIAGEN noncoding sequence: 5'-GGAATCTCATTCGATGCATAC-3' (SEQ ID NO: 18)) and 2.5 µg of DNA of pCMV-hygromycin carrying a cDNA of NAMPT gene (SinoBiological HG10990-M).

### Proliferation assay

A time course curve of parental (empty vector transfected cells) and NAMPT both cDNA and shRNA-expressing cells was generated by seeding 103 cells in 2.2 cm bottom-well diameter dishes in triplicate samples. After 24 hours, medium was changed (day 0) and the indicated culture media added. Every 24 hours, cells were fixed and stained with crystal violet 1% (SIGMA C6158-50G). After extensive washing, crystal violet was solubilized in 20% acetic acid (Sigma) and quantified at 595 nm absorbance as a relative measure of cell number (BIORAD iMarkTM Microplate Reader). Values are expressed as the percentage of cell growth of cells growing in the presence of 10% FBS. Zero percent refers to the number of cells at day 0.

### Colony formation assay and clonal heterogeinity analysis

10³ cells were seeded in 10 cm plates, every condition in triplicate. The medium was replaced every 3 days and after 12 days the colonies were fixed and stained with a Crystal violet assay. After extensive-washing, colonies were counted. Values are expressed in number of observed colonies among 10³ seeded cells. To analyze the clonal heterogeneity, 10² random colonies were classified in triplicate depending on its phenotype: Holoclone, Meroclone and Paraclone (Li H. *et al* 2008.; Garcia-Heredia JM, Lucena-Cacace A, Verdugo-Sivianes EM, Perez M, Carnero A. The cargo protein MAP17 (PDZK1IP1) regulates the cancer stem cell pool activating the Notch pathway by abducting NUMB. Clin Cancer Res. 2017 Feb 2. doi: 10.1158/1078-0432.CCR-16-2358. [

### Sphere-forming assay

A total of 5x10³ cells/mL/well were seeded in Ultra-low Attachment Plates containing MammoCultTM Basal Medium (Human) (Stem Cell Tech) supplied with 0.48 mg/mL hydrocortisone, 0.2% heparin solution and 10% MammoCult™ Proliferation Supplement (Human). After 4 days, primary tumorspheres were measured, both in size and in number, using an inverted microscope (Olympus CKX41).

### Single cell Sphere-forming assay

Single cells were individually seeded through cell sorting (BD FACS Jazz) in 96 well Ultra-low attachment plates containing MammoCultTM Basal medium (Human) (Stem Cell Tech) supplied with hydrocortisone 0.48 mg/mL, Heparin solution 0.2% and 10% MammoCult™ Proliferation Supplement (Human). After 21 days, primary tumorspheres formed were measured both in size and number using inverted microscope (Olympus CKX41). Fluorescence-activated cell sorting (FAC). For FACS staining, live cells were incubated with CD133 (AC133-PE, Miltenyi) and CD44 (PE, Miltenyi) for 30 minutes at dilutions specified in the manufacturer's protocols.

### Quantitative RT-PCR

Total cellular RNA was isolated with the RNeasy kit (Qiagen) and reversed transcribed into cDNA using 2.0 µg of RNA, random primers, dNTP mix and a Multiscribe Reverse Transcriptase in a total volume of 50 µL (High Capacity Transcription Kit-Applied Biosystems). Real time PCR was performed on an Applied Biosystem 7900HT cycler using gene-specific probes from Life technologies as follows: NAMPT (Hs00237184_m1), SNAI1 (Hs00195591_m1), TWIST1 (Hs01675818_s1), FOXC2 (Hs00270951_s1), VIM (Hs00185584m1), NANOG (Hs04260366_g1), BMI-1 (Hs00995536_m1), SOX2 (Hs01053049_s1), OCT4 (Hs00999632_g1), SERPINE1 (Hs00167155_m1), CD133 (Hs01009257_m1), ABCC3 (Hs00978452_m1), CSNK1A1 (Hs00793391_m1), HES1 (Hs00172878_m1), TEAD4 (Hs01125032_m1), c-JUN (Hs01103582_s1), CD44 (Hs01075864_m1) and GADPH (Hs03929097_g1) as housekeeping. Relative quantitation values were expressed as 2-ACt or relative mRNA expression.

### Immunoblotting

Cells were were lysed in RIPA buffer (Tris-HCl pH 8.0 25 mM, NaCl 150 mM, NP40 1%, Sodium desoxycholate 1%, SDS 1%, Na₃VO₄ 1 mM, EDTA 0.5 M, complete protease and phosphatase inhibitor cocktail, 2 mM) and then subjected to 3 sonication cycles during 5 seconds. The amount of protein was determined by Bradford assay using BSA (bovine serum albumin) as a standard. The primary antibodies were purchased from commercial sources as follows: Anti-Visfatin antibody 1:2500 (BETHYL A300-779A), α-tubulin 1:10000 (SIGMA - T9026), The secondary antibodies used were: Goat pAB to Rabbit IgG (HRP) 1:5000 (ABCAM ab97051), Rabbit pAB to Mouse IgG (HRP) 1:5000 (ABCAM ab97046).

### Cytotoxic Assay MTT

5x10³ SF268 cells were seeded then treated with FK866 24 hours later. After 96 hours, cell viability was measured with MTT.

### Retrospective Analysis of NAMPT Gene Expression in Human Gliomas

Correlations between glioma grade, patient survival, tumor recurrence and NAMPT gene expression were determined through analysis of French, TGCA, French-Core Exon, Sun Brain and Freije datasets respectively which are available through Oncomine (Rhodes DR, Yu J, Shanker K, et al. ONCOMINE: A Cancer Microarray Database and Integrated Data-Mining Platform. Neoplasia (New York, NY). 2004;6(1):1-6.) and R2: Genomics analysis and visualization platform (http://r2.amc.nl/). High and low groups were defined as above and below the mean respectively. For analysis with high and low groups, high was defined as greater than one standard deviation above the mean, low is greater than one standard deviation below the mean. The National Cancer Institute's Repository for Molecular Brain Neoplasia Data (REMBRANDT, http://rembrandt.nci.nih.gov) was also evaluated for correlations between glioma patient survival and gene expression with up- or downregulation being defined as a 2 fold change relative to mean values.

### Statistical analysis

All grouped data are presented as mean ± standard error. Difference between groups was assessed by ANOVA or Student's t-test using GraphPad Prism software. For survival analysis, Kaplan Meier curves were generated using Prism software and R2 Kaplan Meier plotting service and log Rank analysis performed. All experiments were repeated in each condition in at least duplicate with triplicate technical replicates. Data distribution was assumed to be normal but this was not formally tested. Data obtained for retrospective analysis were collected and processed in appropriate experimental arms.

### II. RESULTS

### NAMPT Correlates with Glioma Tumor Clinical Outcomes

To confirm the clinical significance of NAMPT expression in brain tumors, publicly available ectoderm-derived tumor datasets for NAMPT levels (Figure 1A) were analyzed. It was determined that gliomas, pilocytic astrocytomas and neuroblastomas express more NAMPT than healthy human brain tissue. We found that NAMPT was highly overexpressed in glioblastoma tissue compared with healthy brain tissue (Figure 1B). This correlation was also observed in an mRNA pool of nine different glioma cell lines (Figure 1 C), as well as in astrocytoma, glioma grade III, glioblastoma, and glioma grade IV tumors (Figure 1C). Furthermore, NAMPT expression was strongly correlated with tumor grade in two independent datasets (Figure 1D-E), indicating that NAMPT is highly overexpressed in human brain tumors and correlates with tumor stage. According to the TCGA molecular classification of Glioblastoma, it was found that NAMPT is particularly expressed in the Classical and Mesenchymal subtype (Figure 1 F).

To further evaluate the potential correlation between NAMPT expression and patient outcome, Kaplan-Meier survival curves were generated from several public databases (Figure 2). In all datasets analyzed, high NAMPT expression was indicative of poor survival (Figure 2A-C). Because NAMPT is closely associated with tumor stage, patients were segregated by tumor grade to determine whether the effects of the enzyme on survival were stage-related or stage-independent. NAMPT correlated with poor prognosis independently of tumor grade in all datasets (Figure 2D-F). It was also found that glioblastoma grade IV tumors expressing high levels of NAMPT had a worse prognosis (Figure 2G), which was confirmed in other datasets (Figure 2H-I). Altogether, these data demonstrate that NAMPT expression in gliomas is an independent indicator of poor patient outcomes, which may indicate that NAMPT has an important oncogenic function in glioma cells that is related to CICs as this subpopulation of cells has been linked to therapy resistance and poor outcomes in tumors.

### NAMPT Strengthens Tumorigenic Properties Enriching Cancer Initiating Cell Phenotype

To elucidate the causal role of NAMPT, it was studied whether NAMPT promotes tumorigenicity in glioma cells. We ectopically expressed NAMPT cDNA in the human glioblastoma cell lines SF268 and U251 MG, then selected transfectants to create a stable transfection pool (NAMPT) (Figure 3A-D). As proof of concept, it was also counteracted endogenous NAMPT gene expression by expressing a small hairpin RNA against NAMPT (sh), producing a daughter cell line expressing reduced levels of the enzyme (Figure 3C-D). The behavior of the parental cell line (expressing only vector, V) with that of isogenic daughter cells exhibiting NAMPT overexpression (NAMPT) or NAMPT underexpression (sh) were then compared. The NAMPT-overexpressing cells grew faster than the parental cells, indicating that NAMPT confers a proliferative advantage (Figure 3E-F), while the NAMPT-underexpressing cells grew slower than the parental cells, confirming the role of NAMPT in tumor proliferation. The NAMPT overexpressing cells also overcame apoptosis when clonogenic assays were performed at low cell densities (Figure 3G-H), while the NAMPT-underexpressing cells formed reduced numbers of colonies compared with the parental cells (Figure 3G-H).

NAMPT overexpression strongly correlated with poor patient prognoses (Figure 2). It has been proposed that CICs are primarily responsible for tumor relapses and poor therapeutic responses, as CICs are able to reconstitute entire tumors.

Therefore, the ability of cells with different levels of NAMPT expression to form tumorspheres was tested, a surrogate assay for the cancer stem-like phenotype of tumorspheres derived from the cells with increased NAMPT expression was significantly higher than that derived from the control and parental cells (Figure 3I), while the cells with decreased NAMPT expression showed a clear reduction in the number of tumorspheres (Figure 3I).

To further explore the cancer-initiating cell properties induced by NAMPT, cells at low densities to form independent colonies comprising individual clones, which were previously classified as holoclones, meroclones and paraclones based on their ability to reconstitute tumors from a single cell, were cultured. Holoclones are believed to be derived from stem cells, while paraclones are differentiated cells that are incapable of reconstituting a culture. Meroclones are intermediate phenotypes between holo- and paraclones. The percentage of holoclones in NAMPT-expressing cells was increased from 20% to 60% in SF268 (Figure 3J-K) and from 60% to 80% in U251MG, while the percentage of holoclones was decreased in cells expressing NAMPT shRNA (Figure 3J-K), indicating a relationship between NAMPT and the CIC phenotype.

In addition, FACS analysis of tumor cells showed higher stem cell phenotypic marker expression, which correlated with NAMPT expression. NAMPT-expressing cells showed increases in both CD133+ (Figure 3L) and CD44+ pools (Figure 3M). However, NAMPT-underexpressing cells showed clear reductions in the CD133+ and CD44+ pools (Figure 3L-M), confirming that NAMPT is a factor that facilitates tumor cell dedifferentiation to a pluripotent-like state. A second shRNA against NAMPT showed similar results (Figure 4 A-C).

To finally confirm these data, the ability of cells with different levels of NAMPT expression to form tumorspheres was tested from one isolated single cell, another surrogate assay for the cancer stem-like phenotype. The cells were seeded at one cell per well and visualized 21 days later (Figure 3N). The parental cells formed spheres at this stage, which were considered 1st generation tumorspheres. The number and size of tumorspheres derived from the cells with increased NAMPT expression was significantly higher than that derived from the control and parental cells (Figure 3O-Q). It was also explored the effect of a second sh in both cell lines in order to avoid overlapping effects and we found similar results (Figure 5).

NAMPT catalyzes the conversion of nicotinamide to nicotinamide mononucleotide (NMN), which is the rate-limiting step in the NAD salvage pathway. If the enzymatic activity of NAMPT is directly responsible for these phenotypes, the phenotype should be recovered by directly adding the product of the enzyme to cells. Therefore, we repeated the previous surrogate experiments and added saturating concentrations of NMN to cells expressing low levels of NAMPT (sh supplemented with NMN, sh+NMN). In these experiments, we rescued the parental phenotypes by supplying the cells with the NAMPT metabolic product NMN (Figure 6A-D left). In all cases, the cells with downregulated NAMPT expression but with abundant NMN in the media behaved similarly to the parental cells. However, they did not exhibit the increased levels of tumorigenicity induced by NAMPT overexpression (Figure 6A-D Left). Similar results were found using a second shRNA against NAMPT (Figure 4A, B).

NAMPT is also known to act as an extracellular soluble protein named visfatin, which has activity that is not related to the enzymatic activity of the protein.

Therefore, the effects of extracellular visfatin supplementation in NAMPT underexpressing cells were explored and found that the NAMPT-underexpressing cells were rescued, similar to the above findings (Figure 6A-D right). However, as before, the cells did not exhibit the increased levels of tumorigenicity induced by NAMPT overexpression (Figure 6A-D right). Similar results were found using a second shRNA against NAMPT (Figure 4A, B).

These results clearly indicate that NAMPT gene overexpression provides the protein with properties beyond those associated with its enzymatic function in the salvage pathway, conferring CIC-like properties to the cells in which is expressed.

### NAMPT Induces Pluripotency via Signalling Pathways Controlling stemness

To support this novel finding, it was decided to explore the correlation between NAMPT expression and cell stemness. Whether the stem transcriptional core formed by SOX2, OCT4 and NANOG was altered by NAMPT levels was analyzed. By Q-RT-PCR, each transcript individually was analyzed, it was confirmed the overexpression of NAMPT in SF268 and U251MG cells (Figure 7A) and found that NAMPT-overexpressing cells significantly induced Sox2, Oct4 and Nanog mRNA expression (Figure 7A). Furthermore, these cells also showed increased levels of CD44 and CD133 transcripts (Figure 7A).

The epithelial-mesenchymal transition (EMT) is an essential step mediating tumor reprogramming and metastasis. Several genes are expressed during EMT induction. For the next step in the study, it was tested whether NAMPT upregulated some of these steps. It was found that NAMPT-overexpressing cells showed induction of FOXC2, TWIST1, VIM, SNAI1 and MMP9 expression (Figure 7B).

Then, iPSC transcriptional databases were analyzed. It was found that NAMPT is highly overexpressed in iPSCs and embryonic stem cells (Figure 7C-E), as they maintain a pluripotent, self-renewed state.

With the aim of identifying the pathways that induce cell stemness and pluripotency, NAMPT expression was correlated with stem cell pathways in several in silico glioma retrospective studies (GSE16011, GSE4290, GSE4271, GSE43378, and GSE7696). A positive correlation with the different stem cell signaling pathways was found. NAMPT expression showed a strong correlation (pearson r) with Hippo, Wnt, Hedgehog and Notch, as well as ABC transporters as markers of stem functionality (Figures 6A-C) (Table 2 and Figure 9). Furthermore, NAMPT also showed correlation to OSKM reprogramming factors (Figure 8C). The analysis of the geneset comprising all these genes that correlate to NAMPT was highly predictive of patient prognosis (Figure 8D). Therefore, it was elected to determine whether the different pathways were hyperactive in NAMPT-overexpressing cells. To this end, Q-RT-PCR to measure individually the mRNA levels for Nampt, CD44, Jun, TEAD4, CSNK1A1, ABCC3, Serpine1 and HES1 was used, as the transcriptional markers of the CSC pathways. Clear transcriptional activation of all these genes in our cells overexpressing NAMPT was observed (Figure 8E). It appears that NAMPT overexpression activates stem cell signaling, eventually activating Hippo, Hedgehog, Wnt and Notch as effectors, contributing to cell stemness.

### NAMPT Triggers a Gene Signature which Correlates with Poor Survival in Glioma

NAMPT induces genes associated with the EMT, the Notch pathway and iPSCs, which increase tumorigenicity and expression of the CIC-like phenotype. It is possible that these factors can be used to predict the prognoses of glioma patients. We selected NAMPT and genes activated by its overexpression, including Jun, CD44, HES1, TEAD4, CSNK1A1, ABCC3 and Serpine1 (Figure 10A). All these transcripts were increased in glioblastoma patient tissue samples compared to their corresponding levels in normal brain tissue (Figure 10B). This signature is able to stratify patients with grade IV glyoblastoma (Figure 10C). Interestingly, this signature also stratify patients with different glioblastoma subtypes according to TCGA database, showing higher expression among mesenchimal subtypes, and lower among the proneural subtypes (Figure 10D). The application of the signature clearly distinguishes the patients with good from bad pronosis in the databases applied (Figures 10E-F).

Through the analysis of the NAMPT-derived signature in different datasets, we observed that the incidence of the gene signature was driven by grade in brain tumor patients (Figure 10C-D and Figure 11A). The percentage of patients who showed a clear positive NAMPT-derived signature increased along with tumor grade in the different datasets analyzed (Figure 12), exceeding 50% among patients with grade IV tumors. Furthermore, comparisons of Kaplan-Meier plots between the groups with grade III and IV tumors with high expression of the signature and the groups with grade IIII and IV tumors with low expression of the signature showed that patients with a positive signature have a poor prognosis (Figure 12). Therefore, the NAMPT-derived signature is capable of accurately predicting poor patient outcomes independently of tumor grade.

Finally, the correlation of our NAMPT-derived signature with other proposed mutations that have been proposed to be drivers of the malignant status of glyoblastoma tumors was analyzed. It was found that the signature of the invention predict an enrichment of IDH1 mutatins in patients negative for this signature, indicating a better prognosis in patients with IDH1 mutations (Figure 11B). On the other hand, most, if not all, glyomas with EGFR amplifications showed a strong positive correlation with our NAMPT-derived signature (Figure 11C). Therefore, it was confirmed that NAMPT is a potent oncogene that confers CIC-like properties and pluripotency, which are responsible for the poorer responses and patient prognoses associated with glioblastoma.

### NAMPT is a suitable target on Glioma CICs

NAMPT is essential for biosynthesis of NAD. Inhibition of NAMPT may lead to depletion of NAD+, which in turn inhibits ATP synthesis. This effect eventually causes attenuation of cancer cell proliferation and death. Therefore, NAMPT has been proposed as an interesting therapeutic target. Our data support this idea, particularly with respect to brain tumor patients with a NAMPT signature, as these patients have poor prognoses.

FK866, a NAMPT inhibitor (Hasmann, M. and Schemainda, I., 2003. Cancer Res 63(21): 7436-7442), was tested as a possible therapy for gliomas in mass cultures and tumorspheres representing CIC populations. NAMPT-overexpressing cells were more sensitive to FK866 in mass cultures than parental cells and NAMPT underexpressing cells. As standard treatment, Temozolomide (TMZ) toxicity was also assessed both individually and in combination with FK866 (Table 2).

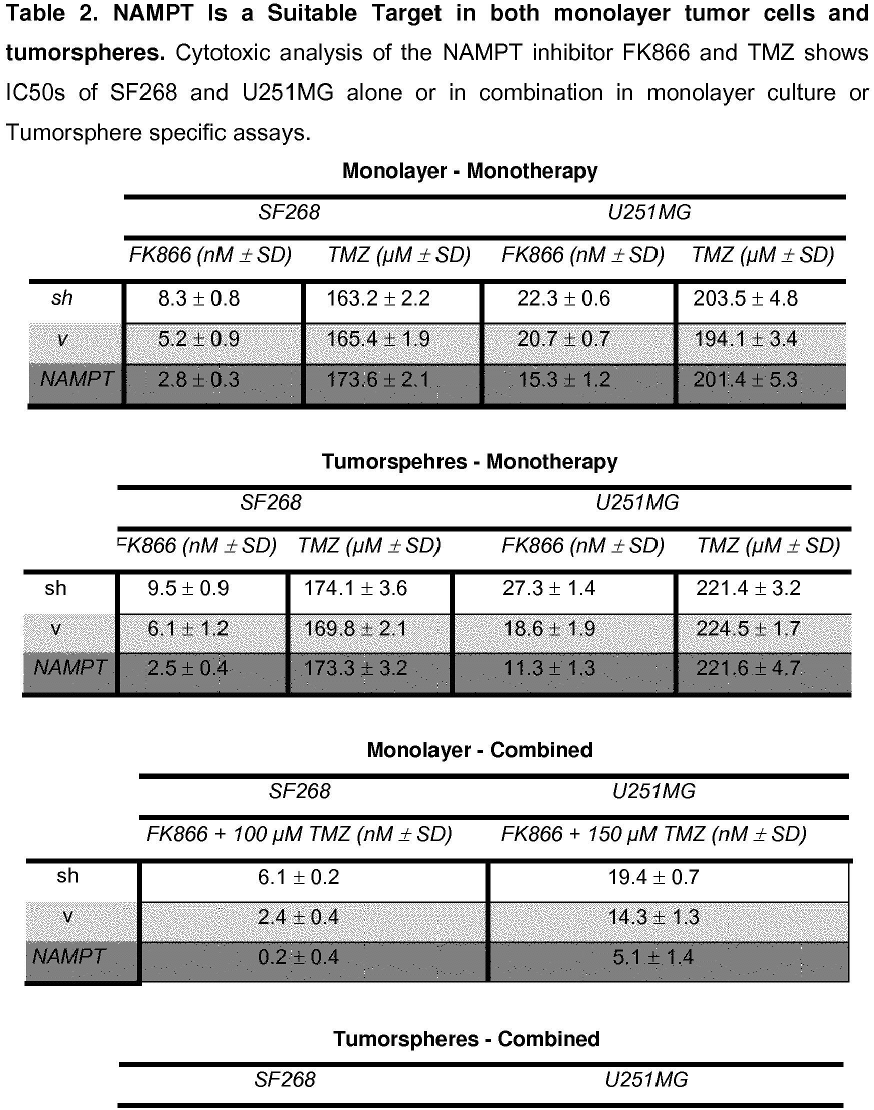

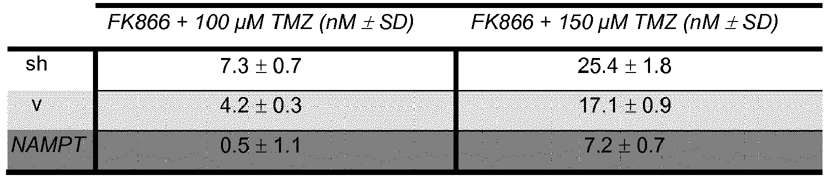

For the assay, 5x10³ cells were seeded and then treated with the different compound at 11 different concentrations at 1/3 dilutions after 24 hours. Then, 96 hours later, cell viability was measured via MTT assay and validated independently by crystal violet staining. IC50 was calculated as the concentration allowing 50% survival compared to day 0 controls. Combination experiments were performed testing 11 different concentrations at 1/3 dilutions of FK866 and maintaining same suboptimal concentration of TMZ (as indicated) in all tested points.

It was assessed FK866 induced toxicity in tumorspheres and observed that FK866 was effective against this CIC population (Table 4). Interestingly, the combined treatment with TMZ slightly sensitized these values, both in mass cultures and tumorspheres suggesting this combination as an effective therapy (Table 4). Thus, it is believe that NAMPT inhibitors, in combination with TMZ may represent a new therapy for glioma CIC populations, particularly in patients expressing high levels of the gene signature.

### NAMPT expression correlated with high levels of cancer initiating cell-like cells in glioblastoma directly from patients.

To approach our findings to a more *in vivo* situation, we first took 14 **glioblastoma** tumor samples directly from patients as well as matched non-tumor samples. In these samples we measured the expression of NAMPT and Nanog, as surrogated marker of CIC-like levels of these tumors (Figure 13A). We found a clear increase of NAMPT and Nanog in tumoral vs non-tumoral samples (Figure 13B and 4C). And more importantly, there was a clear correlation (pearson r=0.53, p<0.01) between the expression of Nanog and NAMPT (Figure 13D). These data reinforce the direct relationship between NAMPT and the cancer stem cell like component of glioblastoma tumors.

Then, we took 5 fresh glioblastoma samples from patients (Supplementary Table S1). After tissue disaggregation, we directly measured NAMPT levels by Q-RT-PCR and in parallel seeded 3000 cells to measure the number of tumorspheres formed (Figure 13E). Then we plotted to establish 1 to 1 correlation, between the level of NAMPT and the number of tumorspheres. We found a strong direct correlation of the tumorsphere number and NAMPT levels for each tumor (Figure 13E, pearson r=0.709; p<0,01).

All these data strongly confirm that NAMPT levels correlates with the activation of the cancer stem cell-like phenotype in human glioblastoma tumors.

## Claims

1. An *in vitro* method for diagnosing glioma in a subject, for determining the prognosis of a subject suffering from glioma, or determining the response of a subject to a therapy against glioma, comprising
(a) determining the expression levels of NAMPT gene and, additionally, the expression levels of, at least, two, three, four, five, six or seven genes from the list consisting of CD44, Jun, TEAD4, CSNK1A1, ABCC3, Serpine1 and HES1 in an isolated biological sample from the subject, and
(b) comparing the expression levels obtained in step (a) with a reference value for each gene,
wherein if the expression levels of said genes are increased with respect to the reference value for each gene, then said subject suffers from glioma, said subject has a poor prognosis, or said subject is not responding to the therapy.

2. Method according to claim 1, wherein therapy against glioma is selected from the group consisting of a NAMPT inhibitor, an angiogenesis inhibitor, preferably an VEGF inhibitor, Opdivo, Rintega, Temozolomide (TMZ), ICT-107, Avastin, ABT-414 in combination with TMZ, ITK-1, Toca511 in combination with Toca-FC, Avastin in combination with VB-111, Veliparib in combination with TMZ, DCVax-L in combination with TMZ, and combinations thereof.

3. Method according to claim 1 or 2, wherein the NAMPT inhibitor is selected from the group consisting of GNE-617, OT-82, MPC-8640, MPC-9528 and combinations thereof.

4. Method according to any one of claims 1 to 3, wherein the glioma is selected from the list consisting of grade I, grade II, grade III and grade IV glioma.

5. Method according to any one of claims 1 to 4, wherein determining the gene expression levels comprises quantifying the messenger RNA (mRNA) of said genes, or a fragment of said mRNA, the complementary DNA (cDNA) of said genes, or a fragment of said cDNA.

6. Method according to claim 5, wherein the expression level is quantified by means of quantitative polymerase chain reaction (QPCR), Real-Time PCR (RT-qPCR), Retro-Transcriptase polymerase chain reaction (RT-PCR), a DNA array, a, RNA array or nucleotide hybridization technique.

7. Method according to any one of claims 1 to 4, wherein determining the expression level of said genes comprises quantifying the levels of protein encoded by said genes or of a functionally equivalent variant of said protein.

8. Method according to claim 7, wherein the levels of protein are quantified by means of western blot, ELISA, immunohistochemistry or a protein array.

9. Method according to any one of claims 1 to 8, wherein the biological sample is selected from the group consisting of blood, serum and brain tissue.

10. A kit or device for in vitro diagnosing glioma in a subject or for determining the prognosis of a subject suffering from glioma, or for determining the response of a subject to a therapy against glioma, comprising
- reagents for specifically determining the expression level of the NAMPT gene, and
- reagents for specifically determining the expression level of, at least, two, three, four, five, six or seven genes selected from the group consisting of CD44, Jun, TEAD4, CSNK1A1, ABCC3, Serpine1 and HES1,
wherein said reagents are selected from the group consisting of probes, primers and/or antibodies specific for each of said genes or the corresponding proteins.

11. Use *in vitro* of a kit or device according to claim 10 for in vitro diagnosing glioma in a subject, or for determining the prognosis of a subject suffering from glioma, or for determining the response of a subject to a therapy against glioma.

12. Use *in vitro* of the expression levels of NAMPT gene and, additionally, the expression levels of, at least, two, three, four, five, six or seven genes from the list consisting of CD44, Jun, TEAD4, CSNK1A1, ABCC3, Serpine1 and HES1 for diagnosing glioma in a subject, for determining the prognosis of a subject suffering from glioma, or determining the response of a subject to a therapy against glioma.

13. A product selected from the list consisting of a NAMPT inhibitor, an angiogenesis inhibitor, preferably an VEGF inhibitor, Opdivo, Rintega, Temozolomide (TMZ), ICT-107, Avastin, ABT-414 in combination with TMZ, ITK-1, Toca511 in combination with Toca-FC, Avastin in combination with VB-111, Veliparib in combination with TMZ, DCVax-L in combination with TMZ, and combinations thereof, for use in the treatment of a subject who has been diagnosed by a method according to any one of claims 1 to 9 as suffering from glioma.

14. A product for use in the treatment of a subject suffering from glioma according to claim 13, wherein the NAMPT inhibitor is selected from the group consisting of GNE-617, OT-82, MPC-8640, MPC-9528 and combinations thereof.

15. A computer program comprising program instructions for causing a computer or a device according to claim 10 or 11 to carry out the method according to any one of claims 1 to 9.


SEQUENCE LISTING

<110>  CONSEJO SUPERIOR DE INVESTIGACIONES CIENTÍFICAS
       SERVICIO ANDALUZ DE SALUD
       UNIVERSIDAD DE SEVILLA

<120>  Expression signature for glioma diagnosis and/or prognosis in a
       subject

<130>  EP1641.1296

<160>  18

<170>  PatentIn version 3.5

<210>  1
<211>  4593
<212>  DNA
<213>  Homo sapiens

<400>  1
gctgccgcgc cccgcccttt ctcggccccc ggagggtgac ggggtgaagg cgggggaacc      60

gaggtgggga gtccgccaga gctcccagac tgcgagcacg cgagccgccg cagccgtcac     120

ccgcgccgcg tcacggctcc cgggcccgcc ctcctctgac ccctcccctc tctccgtttc     180

cccctctccc cctcctccgc cgaccgagca gtgacttaag caacggagcg cggtgaagct     240

catttttctc cttcctcgca gccgcgccag ggagctcgcg gcgcgcggcc cctgtcctcc     300

ggcccgagat gaatcctgcg gcagaagccg agttcaacat cctcctggcc accgactcct     360

acaaggttac tcactataaa caatatccac ccaacacaag caaagtttat tcctactttg     420

aatgccgtga aaagaagaca gaaaactcca aattaaggaa ggtgaaatat gaggaaacag     480

tattttatgg gttgcagtac attcttaata agtacttaaa aggtaaagta gtaaccaaag     540

agaaaatcca ggaagccaaa gatgtctaca aagaacattt ccaagatgat gtctttaatg     600

aaaagggatg gaactacatt cttgagaagt atgatgggca tcttccaata gaaataaaag     660

ctgttcctga gggctttgtc attcccagag gaaatgttct cttcacggtg gaaaacacag     720

atccagagtg ttactggctt acaaattgga ttgagactat tcttgttcag tcctggtatc     780

caatcacagt ggccacaaat tctagagagc agaagaaaat attggccaaa tatttgttag     840

aaacttctgg taacttagat ggtctggaat acaagttaca tgattttggc tacagaggag     900

tctcttccca agagactgct ggcataggag catctgctca cttggttaac ttcaaaggaa     960

cagatacagt agcaggactt gctctaatta aaaaatatta tggaacgaaa gatcctgttc    1020

caggctattc tgttccagca gcagaacaca gtaccataac agcttggggg aaagaccatg    1080

aaaaagatgc ttttgaacat attgtaacac agttttcatc agtgcctgta tctgtggtca    1140

gcgatagcta tgacatttat aatgcgtgtg agaaaatatg gggtgaagat ctaagacatt    1200

taatagtatc aagaagtaca caggcaccac taataatcag acctgattct ggaaaccctc    1260

ttgacactgt gttaaaggtt ttggagattt taggtaagaa gtttcctgtt actgagaact 1320

caaagggtta caagttgctg ccaccttatc ttagagttat tcaaggggat ggagtagata 1380

ttaatacctt acaagagatt gtagaaggca tgaaacaaaa aatgtggagt attgaaaata 1440

ttgccttcgg ttctggtgga ggtttgctac agaagttgac aagagatctc ttgaattgtt 1500

ccttcaagtg tagctatgtt gtaactaatg gccttgggat taacgtcttc aaggacccag 1560

ttgctgatcc caacaaaagg tccaaaaagg gccgattatc tttacatagg acgccagcag 1620

ggaattttgt tacactggag gaaggaaaag gagaccttga ggaatatggt caggatcttc 1680

tccatactgt cttcaagaat ggcaaggtga caaaaagcta ttcatttgat gaaataagaa 1740

aaaatgcaca gctgaatatt gaactggaag cagcacatca ttaggcttta tgactgggtg 1800

tgtgttgtgt gtatgtaata cataatgttt attgtacaga tgtgtggggt ttgtgtttta 1860

tgatacatta cagccaaatt atttgttggt ttatggacat actgcccttt catttttttt 1920

cttttccagt gtttaggtga tctcaaatta ggaaatgcat ttaaccatgt aaaagatgag 1980

tgctaaagta agctttttag ggccctttgc caataggtag tcattcaatc tggtattgat 2040

cttttcacaa ataacagaac tgagaaactt ttatatataa ctgatgatca cataaaacag 2100

atttgcataa aattaccatg attgctttat gtttatattt aacttgtatt tttgtacaaa 2160

caagattgtg taagatatat ttgaagtttc agtgatttaa cagtctttcc aacttttcat 2220

gatttttatg agcacagact ttcaagaaaa tacttgaaaa taaattacat tgccttttgt 2280

ccattaatca gcaaataaaa catggcctta acaaagttgt ttgtgttatt gtacaatttg 2340

aaaattatgt cgggacatac cctatagaat tactaacctt actgcccctt gtagaatatg 2400

tattaatcat tctacattaa agaaaataat ggttcttact ggaatgtcta ggcactgtac 2460

agttattata tatcttggtt gttgtattgt accagtgaaa tgccaaattt gaaaggcctg 2520

tactgcaatt ttatatgtca gagattgcct gtggctctaa tatgcacctc aagattttaa 2580

ggagataatg tttttagaga gaatttctgc ttccactata gaatatatac ataaatgtaa 2640

aatacttaca aaagtggaag tagtgtattt taaagtaatt acacttctga atttattttt 2700

catattctat agttggtatg acttaaatga attactggag tgggtagtga gtgtacttaa 2760

atgtttcaat tctgttatat tttttattaa gtttttaaaa aattaaattg gatattaaat 2820

tgtatggaca tcatttatta attttaaact gaatgccctc aataagtaat actgaagcac 2880

attcttaaat gaagataaat tatctccaat gaaaagcatg acatgtgttt caatagaaga 2940

atcttaagtt ggctaaattc aaagtgcttg acatcaaaat gttctagagt gattagctac 3000

tagattctga atcatacatc acatctgact agagaccagt ttctttcgaa tgattctttt 3060

atgtatgtag atctgttctt ctgaggcagc ggttggccaa ctatagccca aaggccaaat 3120

ttggacttct ttttataaat gcagattgtc tatggctgct ttcccactac tccagcctaa 3180

ggtaaacagc tgcaatagaa gccaaatgag aatcgcaaag cccaaaatgt ttattaacct 3240

gccctttaca caaaattaca caaaaagttt cctgatctct gttctaagaa aaggagtgtg 3300

ccttgcattt aaaaggaaat gttggtttct agggaaggga ggaggctaaa taattgatac 3360

ggaattttcc tcttttgtct tcttttttct cacttaagaa tccgatactg gaagactgat 3420

ttagaaaagt ttttaacatg acattaaatg tgaaatttta aaaattgaaa agccataaat 3480

catctgtttt aaatagttac atgagaaaat gatcactaga ataacctaat tagaagtgtt 3540

atcttcatta aatgtttttt gtaagtggta ttagaaagaa tatgtttttc agatggttct 3600

ttaaacatgt agtgagaaca ataagcatta ttcactttta gtaagtcttc tgtaatccat 3660

gatataaaat aattttaaaa tgatttttta atgtatttga gtaaagatga gtagtattaa 3720

gaaaaacaca catttcttca caaaatgtgc taaggggcgt gtaaagaatc aaaagaaact 3780

attaccaata atagttttga taatcaccca taattttgtg tttaaacatt gaaattatag 3840

tacagacagt attctctgtg ttctgtgaat ttcagcagct tcagaataga gtttaattta 3900

gaaatttgca gtgaaaaaag ctatctcttt gttcacaacc ataaatcagg agatggagat 3960

taattctatt ggctcttagt cacttggaac tgattaattc tgactttctg tcactaagca 4020

cttggtattt ggccatctcc attctgagca ccaaacggtt aacacgaatg tccactagaa 4080

ctctgctgtg tgtcaccctt aaatcagtct aaatcttcca gacaaaagca aatggcattt 4140

atggatttaa gtcattagat tttcaactga cattaattaa tccctcttga ttgattatat 4200

catcaagtat ttatatctta aataggaggt aggatttctg tgttaagact cttatttgta 4260

ccctataatt aaagtaaaat gttttttatg agtatccctt gttttccctt cttaaattgt 4320

tatcaaacaa tttttataat gaaatctatc ttggaaaatt agaaagaaaa atggcaaggt 4380

atttattgtt ctgtttgcca taatttagaa ctcacactta agtattttgt agttttacat 4440

tcctttttaa cccattcagt ggagaatgtc agcttttctc ccaagttgta tgttaagtct 4500

attctaatat gtactcaaca tcaagttata aacatgtaat aaacatggaa ataaagttta 4560

gctctattag tgaagtgtta aaaaaaaaaa aaa 4593

<210> 2
<211> 1023
<212> DNA
<213> Homo sapiens

<400> 2
atggacaagt tttggtggca cgcagcctgg ggactctgcc tcgtgccgct gagcctggcg 60

cagatcgatt tgaatataac ctgccgcttt gcaggtgtat tccacgtgga gaaaaatggt 120

cgctacagca tctctcggac ggaggccgct gacctctgca aggctttcaa tagcaccttg 180

cccacaatgg cccagatgga gaaagctctg agcatcggat ttgagacctg caggtatggg 240

ttcatagaag ggcacgtggt gattccccgg atccacccca actccatctg tgcagcaaac 300

aacacagggg tgtacatcct cacatccaac acctcccagt atgacacata ttgcttcaat 360

gcttcagctc cacctgaaga agattgtaca tcagtcacag acctgcccaa tgcctttgat 420

ggaccaatta ccataactat tgttaaccgt gatggcaccc gctatgtcca gaaaggagaa 480

tacagaacga atcctgaaga catctacccc agcaacccta ctgatgatga cgtgagcagc 540

ggctcctcca gtgaaaggag cagcacttca ggaggttaca tcttttacac cttttctact 600

gtacacccca tcccagacga agacagtccc tggatcaccg acagcacaga cagaatccct 660

gctaccagac actcacatgg gagtcaagaa ggtggagcaa acacaacctc tggtcctata 720

aggacacccc aaattccaga atggctgatc atcttggcat ccctcttggc cttggctttg 780

attcttgcag tttgcattgc agtcaacagt cgaagaaggt gtgggcagaa gaaaaagcta 840

gtgatcaaca gtggcaatgg agctgtggag gacagaaagc caagtggact caacggagag 900

gccagcaagt ctcaggaaat ggtgcatttg gtgaacaagg agtcgtcaga aactccagac 960

cagtttatga cagctgatga gacaaggaac ctgcagaatg tggacatgaa gattggggtg 1020

taa 1023

<210> 3
<211> 3338
<212> DNA
<213> Homo sapiens

<400> 3
gacatcatgg gctattttta ggggttgact ggtagcagat aagtgttgag ctcgggctgg 60

ataagggctc agagttgcac tgagtgtggc tgaagcagcg aggcgggagt ggaggtgcgc 120

ggagtcaggc agacagacag acacagccag ccagccaggt cggcagtata gtccgaactg 180

caaatcttat tttcttttca ccttctctct aactgcccag agctagcgcc tgtggctccc 240

gggctggtgt ttcgggagtg tccagagagc ctggtctcca gccgcccccg ggaggagagc 300

cctgctgccc aggcgctgtt gacagcggcg gaaagcagcg gtacccacgc gcccgccggg 360

ggaagtcggc gagcggctgc agcagcaaag aactttcccg gctgggagga ccggagacaa 420

gtggcagagt cccggagcga acttttgcaa gcctttcctg cgtcttaggc ttctccacgg 480

cggtaaagac cagaaggcgg cggagagcca cgcaagagaa gaaggacgtg cgctcagctt 540

cgctcgcacc ggttgttgaa cttgggcgag cgcgagccgc ggctgccggg cgccccctcc 600

ccctagcagc ggaggagggg acaagtcgtc ggagtccggg cggccaagac ccgccgccgg 660

ccggccactg cagggtccgc actgatccgc tccgcgggga gagccgctgc tctgggaagt 720

gagttcgcct gcggactccg aggaaccgct gcgcccgaag agcgctcagt gagtgaccgc 780

gacttttcaa agccgggtag cgcgcgcgag tcgacaagta agagtgcggg aggcatctta 840

attaaccctg cgctccctgg agcgagctgg tgaggagggc gcagcgggga cgacagccag 900

cgggtgcgtg cgctcttaga gaaactttcc ctgtcaaagg ctccgggggg cgcgggtgtc 960

ccccgcttgc cagagccctg ttgcggcccc gaaacttgtg cgcgcagccc aaactaacct 1020

cacgtgaagt gacggactgt tctatgactg caaagatgga aacgaccttc tatgacgatg 1080

ccctcaacgc ctcgttcctc ccgtccgaga gcggacctta tggctacagt aaccccaaga 1140

tcctgaaaca gagcatgacc ctgaacctgg ccgacccagt ggggagcctg aagccgcacc 1200

tccgcgccaa gaactcggac ctcctcacct cgcccgacgt ggggctgctc aagctggcgt 1260

cgcccgagct ggagcgcctg ataatccagt ccagcaacgg gcacatcacc accacgccga 1320

cccccaccca gttcctgtgc cccaagaacg tgacagatga gcaggagggc ttcgccgagg 1380

gcttcgtgcg cgccctggcc gaactgcaca gccagaacac gctgcccagc gtcacgtcgg 1440

cggcgcagcc ggtcaacggg gcaggcatgg tggctcccgc ggtagcctcg gtggcagggg 1500

gcagcggcag cggcggcttc agcgccagcc tgcacagcga gccgccggtc tacgcaaacc 1560

tcagcaactt caacccaggc gcgctgagca gcggcggcgg ggcgccctcc tacggcgcgg 1620

ccggcctggc ctttcccgcg caaccccagc agcagcagca gccgccgcac cacctgcccc 1680

agcagatgcc cgtgcagcac ccgcggctgc aggccctgaa ggaggagcct cagacagtgc 1740

ccgagatgcc cggcgagaca ccgcccctgt cccccatcga catggagtcc caggagcgga 1800

tcaaggcgga gaggaagcgc atgaggaacc gcatcgctgc ctccaagtgc cgaaaaagga 1860

agctggagag aatcgcccgg ctggaggaaa aagtgaaaac cttgaaagct cagaactcgg 1920

agctggcgtc cacggccaac atgctcaggg aacaggtggc acagcttaaa cagaaagtca 1980

tgaaccacgt taacagtggg tgccaactca tgctaacgca gcagttgcaa acattttgaa 2040

gagagaccgt cgggggctga ggggcaacga agaaaaaaaa taacacagag agacagactt 2100

gagaacttga caagttgcga cggagagaaa aaagaagtgt ccgagaacta aagccaaggg 2160

tatccaagtt ggactgggtt gcgtcctgac ggcgccccca gtgtgcacga gtgggaagga 2220

cttggcgcgc cctcccttgg cgtggagcca gggagcggcc gcctgcgggc tgccccgctt 2280

tgcggacggg ctgtccccgc gcgaacggaa cgttggactt ttcgttaaca ttgaccaaga 2340

actgcatgga cctaacattc gatctcattc agtattaaag gggggagggg gagggggtta 2400

caaactgcaa tagagactgt agattgcttc tgtagtactc cttaagaaca caaagcgggg 2460

ggagggttgg ggaggggcgg caggagggag gtttgtgaga gcgaggctga gcctacagat 2520

gaactctttc tggcctgcct tcgttaactg tgtatgtaca tatatatatt ttttaatttg 2580

atgaaagctg attactgtca ataaacagct tcatgccttt gtaagttatt tcttgtttgt 2640

ttgtttgggt atcctgccca gtgttgtttg taaataagag atttggagca ctctgagttt 2700

accatttgta ataaagtata taattttttt atgttttgtt tctgaaaatt ccagaaagga 2760

tatttaagaa aatacaataa actattggaa agtactcccc taacctcttt tctgcatcat 2820

ctgtagatac tagctatcta ggtggagttg aaagagttaa gaatgtcgat taaaatcact 2880

ctcagtgctt cttactatta agcagtaaaa actgttctct attagacttt agaaataaat 2940

gtacctgatg tacctgatgc tatggtcagg ttatactcct cctcccccag ctatctatat 3000

ggaattgctt accaaaggat agtgcgatgt ttcaggaggc tggaggaagg ggggttgcag 3060

tggagaggga cagcccactg agaagtcaaa catttcaaag tttggattgt atcaagtggc 3120

atgtgctgtg accatttata atgttagtag aaattttaca ataggtgctt attctcaaag 3180

caggaattgg tggcagattt tacaaaagat gtatccttcc aatttggaat cttctctttg 3240

acaattccta gataaaaaga tggcctttgc ttatgaatat ttataacagc attcttgtca 3300

caataaatgt attcaaatac caaaaaaaaa aaaaaaaa 3338

<210> 4
<211> 1215
<212> DNA
<213> Homo sapiens

<400> 4
gttcgttgca acaaattgat gagcaatgct tttttataat gccaactttg tacaaaaaag 60

ttggcatgta tggtcggaac gagctgattg cccgctacat caagctccgg acagggaaga 120

cccgcaccag gaagcaggtc tccagccaca tccaggtgct ggctcgtcgc aaagctcgcg 180

agatccaggc caagctaaag gaccaggcag ctaaggacaa ggccctgcag agcatggctg 240

ccatgtcgtc tgcacagatc atctccgcca cggccttcca cagtagcatg gccctcgccc 300

ggggccccgg ccgcccagca gtctcagggt tttggcaagg agctttgcca ggccaagccg 360

gaacgtccca tgatgtgaag cctttctctc agcaaaccta tgctgtccag cctccgctgc 420

ctctgccagg gtttgagtct cctgcagggc ccgccccatc gccctctgcg cccccggcac 480

ccccatggca gggccgcagc gtggccagct ccaagctctg gatgttggag ttctctgcct 540

tcctggagca gcagcaggac ccggacacgt acaacaagca cctgttcgtg cacattggcc 600

agtccagccc aagctacagc gacccctacc tcgaagccgt ggacatccgc caaatctatg 660

acaaattccc ggagaaaaag ggtggactca aggatctctt cgaacgggga ccctccaatg 720

ccttttttct tgtgaagttc tgggcagacc tcaacaccaa catcgaggat gaaggcagct 780

ccttctatgg ggtctccagc cagtatgaga gccccgagaa catgatcatc acctgctcca 840

cgaaggtctg ctctttcggc aagcaggtgg tggagaaagt tgagacagag tatgctcgct 900

atgagaatgg acactactct taccgcatcc accggtcccc gctctgtgag tacatgatca 960

acttcatcca caagctcaag cacctccctg agaagtacat gatgaacagc gtgctggaga 1020

acttcaccat cctgcaggtg gtcaccaaca gagacacaca ggagaccttg ctgtgcattg 1080

cctatgtctt tgaggtgtca gccagtgagc acggggctca gcaccacatc tacaggctgg 1140

tgaaagaatg cccaactttc ttgtacaaag ttggcattat aagaaagcat tgcttatcaa 1200

tttgttgcaa cgaac 1215

<210> 5
<211> 1143
<212> DNA
<213> Homo sapiens

<400> 5
gttcgttgca acaaattgat gagcaatgct tttttataat gccaactttg tacaaaaaag 60

ttggcatggc gagtagcagc ggctccaagg ctgaattcat tgtcggaggg aaatataaac 120

tggtacggaa gatcgggtct ggctccttcg gggacatcta tttggcgatc aacatcacca 180

acggcgagga agtggcagtg aagctagaat ctcagaaggc caggcatccc cagttgctgt 240

acgagagcaa gctctataag attcttcaag gtggggttgg catcccccac atacggtggt 300

atggtcagga aaaagactac aatgtactag tcatggatct tctgggacct agcctcgaag 360

acctcttcaa tttctgttca agaaggttca caatgaaaac tgtacttatg ttagctgacc 420

agatgatcag tagaattgaa tatgtgcata caaagaattt tatacacaga gacattaaac 480

cagataactt cctaatgggt attgggcgtc actgtaataa gttattcctt attgattttg 540

gtttggccaa aaagtacaga gacaacagga caaggcaaca cataccatac agagaagata 600

aaaacctcac tggcactgcc cgatatgcta gcatcaatgc acatcttggt attgagcaga 660

gtcgccgaga tgacatggaa tcattaggat atgttttgat gtattttaat agaaccagcc 720

tgccatggca agggctaaag gctgcaacaa agaaaaaaaa atatgaaaag attagtgaaa 780

agaagatgtc cacgcctgtt gaagttttat gtaaggggtt tcctgcagaa tttgcgatgt 840

acttaaacta ttgtcgtggg ctacgctttg aggaagcccc agattacatg tatctgaggc 900

agctattccg cattcttttc aggaccctga accatcaata tgactacaca tttgattgga 960

caatgttaaa gcagaaagca gcacagcagg cagcctcttc cagtgggcag ggtcagcagg 1020

cccaaacccc cacaggcaag caaactgaca aaaccaagag taacatgaaa ggtttctacc 1080

caactttctt gtacaaagtt ggcattataa gaaagcattg cttatcaatt tgttgcaacg 1140

aac 1143

<210> 6
<211> 4716
<212> DNA
<213> Homo sapiens

<400> 6
gttcgttgca acaaattgat gagcaatgct tttttataat gccaactttg tacaaaaaag 60

ttggcaccat ggacgccctg tgcggttccg gggagctcgg ctccaagttc tgggactcca 120

acctgtctgt gcacacagaa aacccggacc tcactccctg cttccagaac tccctgctgg 180

cctgggtgcc ctgcatctac ctgtgggtcg ccctgccctg ctacttgctc tacctgcggc 240

accattgtcg tggctacatc atcctctccc acctgtccaa gctcaagatg gtcctgggtg 300

tcctgctgtg gtgcgtctcc tgggcggacc ttttttactc cttccatggc ctggtccatg 360

gccgggcccc tgcccctgtt ttctttgtca cccccttggt ggtgggggtc accatgctgc 420

tggccaccct gctgatacag tatgagcggc tgcagggcgt acagtcttcg ggggtcctca 480

ttatcttctg gttcctgtgt gtggtctgcg ccatcgtccc attccgctcc aagatccttt 540

tagccaaggc agagggtgag atctcagacc ccttccgctt caccaccttc tacatccact 600

ttgccctggt actctctgcc ctcatcttgg cctgcttcag ggagaaacct ccatttttct 660

ccgcaaagaa tgtcgaccct aacccctacc ctgagaccag cgctggcttt ctctcccgcc 720

tgtttttctg gtggttcaca aagatggcca tctatggcta ccggcatccc ctggaggaga 780

aggacctctg gtccctaaag gaagaggaca gatcccagat ggtggtgcag cagctgctgg 840

aggcatggag gaagcaggaa aagcagacgg cacgacacaa ggcttcagca gcacctggga 900

aaaatgcctc cggcgaggac gaggtgctgc tgggtgcccg gcccaggccc cggaagccct 960

ccttcctgaa ggccctgctg gccaccttcg gctccagctt cctcatcagt gcctgcttca 1020

agcttatcca ggacctgctc tccttcatca atccacagct gctcagcatc ctgatcaggt 1080

ttatctccaa ccccatggcc ccctcctggt ggggcttcct ggtggctggg ctgatgttcc 1140

tgtgctccat gatgcagtcg ctgatcttac aacactatta ccactacatc tttgtgactg 1200

gggtgaagtt tcgtactggg atcatgggtg tcatctacag gaaggctctg gttatcacca 1260

actcagtcaa acgtgcgtcc actgtggggg aaattgtcaa cctcatgtca gtggatgccc 1320

agcgcttcat ggaccttgcc cccttcctca atctgctgtg gtcagcaccc ctgcagatca 1380

tcctggcgat ctacttcctc tggcagaacc taggtccctc tgtcctggct ggagtcgctt 1440

tcatggtctt gctgattcca ctcaacggag ctgtggccgt gaagatgcgc gccttccagg 1500

taaagcaaat gaaattgaag gactcgcgca tcaagctgat gagtgagatc ctgaacggca 1560

tcaaggtgct gaagctgtac gcctgggagc ccagcttcct gaagcaggtg gagggcatca 1620

ggcagggtga gctccagctg ctgcgcacgg cggcctacct ccacaccaca accaccttca 1680

cctggatgtg cagccccttc ctggtgaccc tgatcaccct ctgggtgtac gtgtacgtgg 1740

acccaaacaa tgtgctggac gccgagaagg cctttgtgtc tgtgtccttg tttaatatct 1800

taagacttcc cctcaacatg ctgccccagt taatcagcaa cctgactcag gccagtgtgt 1860

ctctgaaacg gatccagcaa ttcctgagcc aagaggaact tgacccccag agtgtggaaa 1920

gaaagaccat ctccccaggc tatgccatca ccatacacag tggcaccttc acctgggccc 1980

aggacctgcc ccccactctg cacagcctag acatccaggt cccgaaaggg gcactggtgg 2040

ccgtggtggg gcctgtgggc tgtgggaagt cctccctggt gtctgccctg ctgggagaga 2100

tggagaagct agaaggcaaa gtgcacatga agggctccgt ggcctatgtg ccccagcagg 2160

catggatcca gaactgcact cttcaggaaa acgtgctttt cggcaaagcc ctgaacccca 2220

agcgctacca gcagactctg gaggcctgtg ccttgctagc tgacctggag atgctgcctg 2280

gtggggatca gacagagatt ggagagaagg gcattaacct gtctgggggc cagcggcagc 2340

gggtcagtct ggctcgagct gtttacagtg atgccgatat tttcttgctg gatgacccac 2400

tgtccgcggt ggactctcat gtggccaagc acatctttga ccacgtcatc gggccagaag 2460

gcgtgctggc aggcaagacg cgagtgctgg tgacgcacgg cattagcttc ctgccccaga 2520

cagacttcat cattgtgcta gctgatggac aggtgtctga gatgggcccg tacccagccc 2580

tgctgcagcg caacggctcc tttgccaact ttctctgcaa ctatgccccc gatgaggacc 2640

aagggcacct ggaggacagc tggaccgcgt tggaaggtgc agaggataag gaggcactgc 2700

tgattgaaga cacactcagc aaccacacgg atctgacaga caatgatcca gtcacctatg 2760

tggtccagaa gcagtttatg agacagctga gtgccctgtc ctcagatggg gagggacagg 2820

gtcggcctgt accccggagg cacctgggtc catcagagaa ggtgcaggtg acagaggcga 2880

aggcagatgg ggcactgacc caggaggaga aagcagccat tggcactgtg gagctcagtg 2940

tgttctggga ttatgccaag gccgtggggc tctgtaccac gctggccatc tgtctcctgt 3000

atgtgggtca aagtgcggct gccattggag ccaatgtgtg gctcagtgcc tggacaaatg 3060

atgccatggc agacagtaga cagaacaaca cttccctgag gctgggcgtc tatgctgctt 3120

taggaattct gcaagggttc ttggtgatgc tggcagccat ggccatggca gcgggtggca 3180

tccaggctgc ccgtgtgttg caccaggcac tgctgcacaa caagatacgc tcgccacagt 3240

ccttctttga caccacacca tcaggccgca tcctgaactg cttctccaag gacatctatg 3300

tcgttgatga ggttctggcc cctgtcatcc tcatgctgct caattccttc ttcaacgcca 3360

tctccactct tgtggtcatc atggccagca cgccgctctt cactgtggtc atcctgcccc 3420

tggctgtgct ctacacctta gtgcagcgct tctatgcagc cacatcacgg caactgaagc 3480

ggctggaatc agtcagccgc tcacctatct actcccactt ttcggagaca gtgactggtg 3540

ccagtgtcat ccgggcctac aaccgcagcc gggattttga gatcatcagt gatactaagg 3600

tggatgccaa ccagagaagc tgctacccct acatcatctc caaccggtgg ctgagcatcg 3660

gagtggagtt cgtggggaac tgcgtggtgc tctttgctgc actatttgcc gtcatcggga 3720

ggagcagcct gaacccgggg ctggtgggcc tttctgtgtc ctactccttg caggtgacat 3780

ttgctctgaa ctggatgata cgaatgatgt cagatttgga atctaacatc gtggctgtgg 3840

agagggtcaa ggagtactcc aagacagaga cagaggcgcc ctgggtggtg gaaggcagcc 3900

gccctcccga aggttggccc ccacgtgggg aggtggagtt ccggaattat tctgtgcgct 3960

accggccggg cctagacctg gtgctgagag acctgagtct gcatgtgcac ggtggcgaga 4020

aggtggggat cgtgggccgc actggggctg gcaagtcttc catgaccctt tgcctgttcc 4080

gcatcctgga ggcggcaaag ggtgaaatcc gcattgatgg cctcaatgtg gcagacatcg 4140

gcctccatga cctgcgctct cagctgacca tcatcccgca ggaccccatc ctgttctcgg 4200

ggaccctgcg catgaacctg gaccccttcg gcagctactc agaggaggac atttggtggg 4260

ctttggagct gtcccacctg cacacgtttg tgagctccca gccggcaggc ctggacttcc 4320

agtgctcaga gggcggggag aatctcagcg tgggccagag gcagctcgtg tgcctggccc 4380

gagccctgct ccgcaagagc cgcatcctgg ttttagacga ggccacagct gccatcgacc 4440

tggagactga caacctcatc caggctacca tccgcaccca gtttgatacc tgcactgtcc 4500

tgaccatcgc acaccggctt aacactatca tggactacac cagggtcctg gtcctggaca 4560

aaggagtagt agctgaattt gattctccag ccaacctcat tgcagctaga ggcatcttct 4620

acgggatggc cagagatgct ggacttgcct tgccaacttt cttgtacaaa gttggcatta 4680

taagaaagca ttgcttatca atttgttgca acgaac 4716

<210> 7
<211> 1338
<212> DNA
<213> Homo sapiens

<400> 7
gttcgttgca acaaattgat gagcaatgct tttttataat gccaactttg tacaaaaaag 60

ttggcatgca gatgtctcca gccctcacct gcctagtcct gggcctggcc cttgtctttg 120

gtgaagggtc tgctgtgcac catcccccat cctacgtggc ccacctggcc tcagacttcg 180

gggtgagggt gtttcagcag gtggcgcagg cctccaagga ccgcaacgtg gttttctcac 240

cctatggggt ggcctcggtg ttggccatgc tccagctgac aacaggagga gaaacccagc 300

agcagattca agcagctatg ggattcaaga ttgatgacaa gggcatggcc cccgccctcc 360

ggcatctgta caaggagctc atggggccat ggaacaagga tgagatcagc accacagacg 420

cgatcttcgt ccagcgggat ctgaagctgg tccagggctt catgccccac ttcttcaggc 480

tgttccggag cacggtcaag caagtggact tttcagaggt ggagagagcc agattcatca 540

tcaatgactg ggtgaagaca cacacaaaag gtatgatcag caacttgctt gggaaaggag 600

ccgtggacca gctgacacgg ctggtgctgg tgaatgccct ctacttcaac ggccagtgga 660

agactccctt ccccgactcc agcacccacc gccgcctctt ccacaaatca gacggcagca 720

ctgtctctgt gcccatgatg gctcagacca acaagttcaa ctatactgag ttcaccacgc 780

ccgatggcca ttactacgac atcctggaac tgccctacca cggggacacc ctcagcatgt 840

tcattgctgc cccttatgaa aaagaggtgc ctctctctgc cctcaccaac attctgagtg 900

cccagctcat cagccactgg aaaggcaaca tgaccaggct gccccgcctc ctggttctgc 960

ccaagttctc cctggagact gaagtcgacc tcaggaagcc cctagagaac ctgggaatga 1020

ccgacatgtt cagacagttt caggctgact tcacgagtct ttcagaccaa gagcctctcc 1080

acgtcgcgca ggcgctgcag aaagtgaaga tcgaggtgaa cgagagtggc acggtggcct 1140

cctcatccac agctgtcata gtctcagccc gcatggcccc cgaggagatc atcatggaca 1200

gacccttcct ctttgtggtc cggcacaacc ccacaggaac agtccttttc atgggccaag 1260

tgatagaacc ctgcccaact ttcttgtaca aagttggcat tataagaaag cattgcttat 1320

caatttgttg caacgaac 1338

<210> 8
<211> 1649
<212> DNA
<213> Homo sapiens

<400> 8
gtttgtagag gggtgggtgc gcacgtctgt ccctgcgtga ccttccgacc ccgctgtcct 60

caccgcaatg gcggctgtga gggccctggt ggcctcgagg ctcgctgcgg catctgcatt 120

cacgtccctg tcccccggcg gtcggacgcc ttcccagcgc gcagcccttc acctctccgt 180

gccgcgcccc gcggccaggg tcgcgctggt gctgtctgga tgcggagtct acgatgggac 240

cgagatccac gaggcctcgg cgatcctggt gcacctgagc cgtggagggg ctgaagtcca 300

gatctttgct cctgacgtcc ctcagatgca cgtgattgac cacaccaagg ggcagccgtc 360

cgaaggcgag agcaggaatg ttttgaccga gtctgcgagg atcgcccgtg gcaaaatcac 420

agacctggcc aacctcagtg cagccaacca tgatgctgcc atctttccag gaggctttgg 480

agcggctaaa aacctgagca cgtttgccgt ggacgggaaa gattgcaagg tgaataaaga 540

agtggagcgt gtcctgaagg agttccacca ggccgggaag cccatcggct tgtgctgcat 600

tgcacctgtc ctcgcggcca aggtgctcag aggcgtcgag gtgactgtgg gccacgagca 660

ggaggaaggt ggcaagtggc cttatgccgg gaccgcagag gccatgaagg ccctgggtgc 720

caagcactgc gtgaaggaag tggtcgaagc tcacgtggac cagaaaaaca aggtggtcac 780

gaccccagcc ttcatgtgcg agacggcact ccactacatc catgatggga tcggagccat 840

ggtgaggaag gtgctggaac tcactggaaa gtgacgcgca tggacggggc ccagctaggc 900

gccaggactt ggcctcaccc tctggctgag gagctgtcgg ctgctttcca tccagctggg 960

agtctggcag gccctttttt ttttttcttt gccgaaacct gcaggcgttc tctctctaag 1020

gaggatgtgc tgcagtgcat gggggatgtt tcttcctggg tgtggctggg ctgctctcac 1080

atacagaggc cgaggggcca attcgttctc tgccacaggg acttgcctca ctgtgtccca 1140

aaaacaaatc gcagccagct tttccagaaa tagaaaattc tgccgtctga ggttttatac 1200

ttcaggttag ttagtttttg gaaggaagaa catttttagg tttgcaagcc tcctgatcag 1260

gaaaccagaa ataccacatt tatggaccat gaaaggttgg ttcttgactc tgaagggact 1320

tttgagttaa tcagcgtaag gggatttcta aagcaggcaa tccctgtagc cgcagagaat 1380

aaacgccttc ccaaaatggc aacttcccac agccacattt cagacctgct gagactgctg 1440

agtgaggaat ggcagtgagg tttcttcaat tagtctcagt tctcttaatt ttcaggaaga 1500

aagggaaatt gcagctcctc agcccccagg attgacctct ggggagtgat ggtagcgttg 1560

gtgccaggcc gtgggttcag gtgtggcaga agcttgcaga tgcgtccgaa gggaaataaa 1620

gtgtgttggc gttagacttt gtgctgcaa 1649

<210> 9
<211> 491
<212> PRT
<213> Homo sapiens

<400> 9

<210>  10
<211>  340
<212>  PRT
<213>  Homo sapiens

<400>  10

Met Asp Lys Phe Trp Trp His Ala Ala Trp Gly Leu Cys Leu Val Pro
1               5                   10                  15

Leu Ser Leu Ala Gln Ile Asp Leu Asn Ile Thr Cys Arg Phe Ala Gly
            20                  25                  30

Val Phe His Val Glu Lys Asn Gly Arg Tyr Ser Ile Ser Arg Thr Glu
        35                  40                  45

Ala Ala Asp Leu Cys Lys Ala Phe Asn Ser Thr Leu Pro Thr Met Ala
    50                  55                  60

Gln Met Glu Lys Ala Leu Ser Ile Gly Phe Glu Thr Cys Arg Tyr Gly
65                  70                  75                  80

Phe Ile Glu Gly His Val Val Ile Pro Arg Ile His Pro Asn Ser Ile
                85                  90                  95

Cys Ala Ala Asn Asn Thr Gly Val Tyr Ile Leu Thr Ser Asn Thr Ser
            100                 105                 110

Gln Tyr Asp Thr Tyr Cys Phe Asn Ala Ser Ala Pro Pro Glu Glu Asp

115                 120                 125

Cys Thr Ser Val Thr Asp Leu Pro Asn Ala Phe Asp Gly Pro Ile Thr
    130                 135                 140

Ile Thr Ile Val Asn Arg Asp Gly Thr Arg Tyr Val Gln Lys Gly Glu
145                 150                 155                 160

Tyr Arg Thr Asn Pro Glu Asp Ile Tyr Pro Ser Asn Pro Thr Asp Asp
                165                 170                 175

Asp Val Ser Ser Gly Ser Ser Ser Glu Arg Ser Ser Thr Ser Gly Gly
            180                 185                 190

Tyr Ile Phe Tyr Thr Phe Ser Thr Val His Pro Ile Pro Asp Glu Asp
        195                 200                 205

Ser Pro Trp Ile Thr Asp Ser Thr Asp Arg Ile Pro Ala Thr Arg His
    210                 215                 220

Ser His Gly Ser Gln Glu Gly Gly Ala Asn Thr Thr Ser Gly Pro Ile
225                 230                 235                 240

Arg Thr Pro Gln Ile Pro Glu Trp Leu Ile Ile Leu Ala Ser Leu Leu
                245                 250                 255

Ala Leu Ala Leu Ile Leu Ala Val Cys Ile Ala Val Asn Ser Arg Arg
            260                 265                 270

Arg Cys Gly Gln Lys Lys Lys Leu Val Ile Asn Ser Gly Asn Gly Ala
        275                 280                 285

Val Glu Asp Arg Lys Pro Ser Gly Leu Asn Gly Glu Ala Ser Lys Ser
    290                 295                 300

Gln Glu Met Val His Leu Val Asn Lys Glu Ser Ser Glu Thr Pro Asp
305                 310                 315                 320

Gln Phe Met Thr Ala Asp Glu Thr Arg Asn Leu Gln Asn Val Asp Met
                325                 330                 335

Lys Ile Gly Val
            340

<210>  11
<211>  331
<212>  PRT
<213>  Homo sapiens

<400>  11

Met Thr Ala Lys Met Glu Thr Thr Phe Tyr Asp Asp Ala Leu Asn Ala
1               5                   10                  15

Ser Phe Leu Pro Ser Glu Ser Gly Pro Tyr Gly Tyr Ser Asn Pro Lys
            20                  25                  30

Ile Leu Lys Gln Ser Met Thr Leu Asn Leu Ala Asp Pro Val Gly Ser
        35                  40                  45

Leu Lys Pro His Leu Arg Ala Lys Asn Ser Asp Leu Leu Thr Ser Pro
    50                  55                  60

Asp Val Gly Leu Leu Lys Leu Ala Ser Pro Glu Leu Glu Arg Leu Ile
65                  70                  75                  80

Ile Gln Ser Ser Asn Gly His Ile Thr Thr Thr Pro Thr Pro Thr Gln
                85                  90                  95

Phe Leu Cys Pro Lys Asn Val Thr Asp Glu Gln Glu Gly Phe Ala Glu
            100                 105                 110

Gly Phe Val Arg Ala Leu Ala Glu Leu His Ser Gln Asn Thr Leu Pro
        115                 120                 125

Ser Val Thr Ser Ala Ala Gln Pro Val Asn Gly Ala Gly Met Val Ala
    130                 135                 140

Pro Ala Val Ala Ser Val Ala Gly Gly Ser Gly Ser Gly Gly Phe Ser
145                 150                 155                 160

Ala Ser Leu His Ser Glu Pro Pro Val Tyr Ala Asn Leu Ser Asn Phe
                165                 170                 175

Asn Pro Gly Ala Leu Ser Ser Gly Gly Gly Ala Pro Ser Tyr Gly Ala
            180                 185                 190

Ala Gly Leu Ala Phe Pro Ala Gln Pro Gln Gln Gln Gln Gln Pro Pro
        195                 200                 205

His His Leu Pro Gln Gln Met Pro Val Gln His Pro Arg Leu Gln Ala
    210                 215                 220

Leu Lys Glu Glu Pro Gln Thr Val Pro Glu Met Pro Gly Glu Thr Pro
225                 230                 235                 240

Pro Leu Ser Pro Ile Asp Met Glu Ser Gln Glu Arg Ile Lys Ala Glu
                245                 250                 255

Arg Lys Arg Met Arg Asn Arg Ile Ala Ala Ser Lys Cys Arg Lys Arg
            260                 265                 270

Lys Leu Glu Arg Ile Ala Arg Leu Glu Glu Lys Val Lys Thr Leu Lys
        275                 280                 285

Ala Gln Asn Ser Glu Leu Ala Ser Thr Ala Asn Met Leu Arg Glu Gln
    290                 295                 300

Val Ala Gln Leu Lys Gln Lys Val Met Asn His Val Asn Ser Gly Cys
305                 310                 315                 320

Gln Leu Met Leu Thr Gln Gln Leu Gln Thr Phe
                325                 330

<210>  12
<211>  361
<212>  PRT
<213>  Homo sapiens

<400>  12

Met Tyr Gly Arg Asn Glu Leu Ile Ala Arg Tyr Ile Lys Leu Arg Thr
1               5                   10                  15

Gly Lys Thr Arg Thr Arg Lys Gln Val Ser Ser His Ile Gln Val Leu
            20                  25                  30

Ala Arg Arg Lys Ala Arg Glu Ile Gln Ala Lys Leu Lys Asp Gln Ala
        35                  40                  45

Ala Lys Asp Lys Ala Leu Gln Ser Met Ala Ala Met Ser Ser Ala Gln
    50                  55                  60

Ile Ile Ser Ala Thr Ala Phe His Ser Ser Met Ala Leu Ala Arg Gly
65                  70                  75                  80

Pro Gly Arg Pro Ala Val Ser Gly Phe Trp Gln Gly Ala Leu Pro Gly
                85                  90                  95

Gln Ala Gly Thr Ser His Asp Val Lys Pro Phe Ser Gln Gln Thr Tyr
            100                 105                 110

Ala Val Gln Pro Pro Leu Pro Leu Pro Gly Phe Glu Ser Pro Ala Gly
        115                 120                 125

Pro Ala Pro Ser Pro Ser Ala Pro Pro Ala Pro Pro Trp Gln Gly Arg
    130                 135                 140

Ser Val Ala Ser Ser Lys Leu Trp Met Leu Glu Phe Ser Ala Phe Leu
145                 150                 155                 160

Glu Gln Gln Gln Asp Pro Asp Thr Tyr Asn Lys His Leu Phe Val His
                165                 170                 175

Ile Gly Gln Ser Ser Pro Ser Tyr Ser Asp Pro Tyr Leu Glu Ala Val
            180                 185                 190

Asp Ile Arg Gln Ile Tyr Asp Lys Phe Pro Glu Lys Lys Gly Gly Leu
        195                 200                 205

Lys Asp Leu Phe Glu Arg Gly Pro Ser Asn Ala Phe Phe Leu Val Lys
    210                 215                 220

Phe Trp Ala Asp Leu Asn Thr Asn Ile Glu Asp Glu Gly Ser Ser Phe
225                 230                 235                 240

Tyr Gly Val Ser Ser Gln Tyr Glu Ser Pro Glu Asn Met Ile Ile Thr
                245                 250                 255

Cys Ser Thr Lys Val Cys Ser Phe Gly Lys Gln Val Val Glu Lys Val
            260                 265                 270

Glu Thr Glu Tyr Ala Arg Tyr Glu Asn Gly His Tyr Ser Tyr Arg Ile
        275                 280                 285

His Arg Ser Pro Leu Cys Glu Tyr Met Ile Asn Phe Ile His Lys Leu
    290                 295                 300

Lys His Leu Pro Glu Lys Tyr Met Met Asn Ser Val Leu Glu Asn Phe
305                 310                 315                 320

Thr Ile Leu Gln Val Val Thr Asn Arg Asp Thr Gln Glu Thr Leu Leu
                325                 330                 335

Cys Ile Ala Tyr Val Phe Glu Val Ser Ala Ser Glu His Gly Ala Gln
            340                 345                 350

His His Ile Tyr Arg Leu Val Lys Glu
        355                 360

<210>  13
<211>  337
<212>  PRT

<213>  Homo sapiens

<400>  13

Met Ala Ser Ser Ser Gly Ser Lys Ala Glu Phe Ile Val Gly Gly Lys
1               5                   10                  15

Tyr Lys Leu Val Arg Lys Ile Gly Ser Gly Ser Phe Gly Asp Ile Tyr
            20                  25                  30

Leu Ala Ile Asn Ile Thr Asn Gly Glu Glu Val Ala Val Lys Leu Glu
        35                  40                  45

Ser Gln Lys Ala Arg His Pro Gln Leu Leu Tyr Glu Ser Lys Leu Tyr
    50                  55                  60

Lys Ile Leu Gln Gly Gly Val Gly Ile Pro His Ile Arg Trp Tyr Gly
65                  70                  75                  80

Gln Glu Lys Asp Tyr Asn Val Leu Val Met Asp Leu Leu Gly Pro Ser
                85                  90                  95

Leu Glu Asp Leu Phe Asn Phe Cys Ser Arg Arg Phe Thr Met Lys Thr
            100                 105                 110

Val Leu Met Leu Ala Asp Gln Met Ile Ser Arg Ile Glu Tyr Val His
        115                 120                 125

Thr Lys Asn Phe Ile His Arg Asp Ile Lys Pro Asp Asn Phe Leu Met
    130                 135                 140

Gly Ile Gly Arg His Cys Asn Lys Leu Phe Leu Ile Asp Phe Gly Leu
145                 150                 155                 160

Ala Lys Lys Tyr Arg Asp Asn Arg Thr Arg Gln His Ile Pro Tyr Arg
                165                 170                 175

Glu Asp Lys Asn Leu Thr Gly Thr Ala Arg Tyr Ala Ser Ile Asn Ala
            180                 185                 190

His Leu Gly Ile Glu Gln Ser Arg Arg Asp Asp Met Glu Ser Leu Gly
        195                 200                 205

Tyr Val Leu Met Tyr Phe Asn Arg Thr Ser Leu Pro Trp Gln Gly Leu
    210                 215                 220

Lys Ala Ala Thr Lys Lys Lys Lys Tyr Glu Lys Ile Ser Glu Lys Lys
225                 230                 235                 240

Met Ser Thr Pro Val Glu Val Leu Cys Lys Gly Phe Pro Ala Glu Phe
                245                 250                 255

Ala Met Tyr Leu Asn Tyr Cys Arg Gly Leu Arg Phe Glu Glu Ala Pro
            260                 265                 270

Asp Tyr Met Tyr Leu Arg Gln Leu Phe Arg Ile Leu Phe Arg Thr Leu
        275                 280                 285

Asn His Gln Tyr Asp Tyr Thr Phe Asp Trp Thr Met Leu Lys Gln Lys
    290                 295                 300

Ala Ala Gln Gln Ala Ala Ser Ser Ser Gly Gln Gly Gln Gln Ala Gln
305                 310                 315                 320

Thr Pro Thr Gly Lys Gln Thr Asp Lys Thr Lys Ser Asn Met Lys Gly
                325                 330                 335

Phe

<210>  14
<211>  1527
<212>  PRT
<213>  Homo sapiens

<400>  14

Met Asp Ala Leu Cys Gly Ser Gly Glu Leu Gly Ser Lys Phe Trp Asp
1               5                   10                  15

Ser Asn Leu Ser Val His Thr Glu Asn Pro Asp Leu Thr Pro Cys Phe
            20                  25                  30

Gln Asn Ser Leu Leu Ala Trp Val Pro Cys Ile Tyr Leu Trp Val Ala
        35                  40                  45

Leu Pro Cys Tyr Leu Leu Tyr Leu Arg His His Cys Arg Gly Tyr Ile
    50                  55                  60

Ile Leu Ser His Leu Ser Lys Leu Lys Met Val Leu Gly Val Leu Leu
65                  70                  75                  80

Trp Cys Val Ser Trp Ala Asp Leu Phe Tyr Ser Phe His Gly Leu Val
                85                  90                  95

His Gly Arg Ala Pro Ala Pro Val Phe Phe Val Thr Pro Leu Val Val
            100                 105                 110

Gly Val Thr Met Leu Leu Ala Thr Leu Leu Ile Gln Tyr Glu Arg Leu
        115                 120                 125

Gln Gly Val Gln Ser Ser Gly Val Leu Ile Ile Phe Trp Phe Leu Cys
    130                 135                 140

Val Val Cys Ala Ile Val Pro Phe Arg Ser Lys Ile Leu Leu Ala Lys
145                 150                 155                 160

Ala Glu Gly Glu Ile Ser Asp Pro Phe Arg Phe Thr Thr Phe Tyr Ile
                165                 170                 175

His Phe Ala Leu Val Leu Ser Ala Leu Ile Leu Ala Cys Phe Arg Glu
            180                 185                 190

Lys Pro Pro Phe Phe Ser Ala Lys Asn Val Asp Pro Asn Pro Tyr Pro
        195                 200                 205

Glu Thr Ser Ala Gly Phe Leu Ser Arg Leu Phe Phe Trp Trp Phe Thr
    210                 215                 220

Lys Met Ala Ile Tyr Gly Tyr Arg His Pro Leu Glu Glu Lys Asp Leu
225                 230                 235                 240

Trp Ser Leu Lys Glu Glu Asp Arg Ser Gln Met Val Val Gln Gln Leu
                245                 250                 255

Leu Glu Ala Trp Arg Lys Gln Glu Lys Gln Thr Ala Arg His Lys Ala
            260                 265                 270

Ser Ala Ala Pro Gly Lys Asn Ala Ser Gly Glu Asp Glu Val Leu Leu
        275                 280                 285

Gly Ala Arg Pro Arg Pro Arg Lys Pro Ser Phe Leu Lys Ala Leu Leu
    290                 295                 300

Ala Thr Phe Gly Ser Ser Phe Leu Ile Ser Ala Cys Phe Lys Leu Ile
305                 310                 315                 320

Gln Asp Leu Leu Ser Phe Ile Asn Pro Gln Leu Leu Ser Ile Leu Ile
                325                 330                 335

Arg Phe Ile Ser Asn Pro Met Ala Pro Ser Trp Trp Gly Phe Leu Val
            340                 345                 350

Ala Gly Leu Met Phe Leu Cys Ser Met Met Gln Ser Leu Ile Leu Gln
        355                 360                 365

His Tyr Tyr His Tyr Ile Phe Val Thr Gly Val Lys Phe Arg Thr Gly
    370                 375                 380

Ile Met Gly Val Ile Tyr Arg Lys Ala Leu Val Ile Thr Asn Ser Val
385                 390                 395                 400

Lys Arg Ala Ser Thr Val Gly Glu Ile Val Asn Leu Met Ser Val Asp
                405                 410                 415

Ala Gln Arg Phe Met Asp Leu Ala Pro Phe Leu Asn Leu Leu Trp Ser
            420                 425                 430

Ala Pro Leu Gln Ile Ile Leu Ala Ile Tyr Phe Leu Trp Gln Asn Leu
        435                 440                 445

Gly Pro Ser Val Leu Ala Gly Val Ala Phe Met Val Leu Leu Ile Pro
    450                 455                 460

Leu Asn Gly Ala Val Ala Val Lys Met Arg Ala Phe Gln Val Lys Gln
465                 470                 475                 480

Met Lys Leu Lys Asp Ser Arg Ile Lys Leu Met Ser Glu Ile Leu Asn
                485                 490                 495

Gly Ile Lys Val Leu Lys Leu Tyr Ala Trp Glu Pro Ser Phe Leu Lys
            500                 505                 510

Gln Val Glu Gly Ile Arg Gln Gly Glu Leu Gln Leu Leu Arg Thr Ala
        515                 520                 525

Ala Tyr Leu His Thr Thr Thr Thr Phe Thr Trp Met Cys Ser Pro Phe
    530                 535                 540

Leu Val Thr Leu Ile Thr Leu Trp Val Tyr Val Tyr Val Asp Pro Asn
545                 550                 555                 560

Asn Val Leu Asp Ala Glu Lys Ala Phe Val Ser Val Ser Leu Phe Asn
                565                 570                 575

Ile Leu Arg Leu Pro Leu Asn Met Leu Pro Gln Leu Ile Ser Asn Leu
            580                 585                 590

Thr Gln Ala Ser Val Ser Leu Lys Arg Ile Gln Gln Phe Leu Ser Gln
        595                 600                 605

Glu Glu Leu Asp Pro Gln Ser Val Glu Arg Lys Thr Ile Ser Pro Gly

610                 615                 620

Tyr Ala Ile Thr Ile His Ser Gly Thr Phe Thr Trp Ala Gln Asp Leu
625                 630                 635                 640

Pro Pro Thr Leu His Ser Leu Asp Ile Gln Val Pro Lys Gly Ala Leu
                645                 650                 655

Val Ala Val Val Gly Pro Val Gly Cys Gly Lys Ser Ser Leu Val Ser
            660                 665                 670

Ala Leu Leu Gly Glu Met Glu Lys Leu Glu Gly Lys Val His Met Lys
        675                 680                 685

Gly Ser Val Ala Tyr Val Pro Gln Gln Ala Trp Ile Gln Asn Cys Thr
    690                 695                 700

Leu Gln Glu Asn Val Leu Phe Gly Lys Ala Leu Asn Pro Lys Arg Tyr
705                 710                 715                 720

Gln Gln Thr Leu Glu Ala Cys Ala Leu Leu Ala Asp Leu Glu Met Leu
                725                 730                 735

Pro Gly Gly Asp Gln Thr Glu Ile Gly Glu Lys Gly Ile Asn Leu Ser
            740                 745                 750

Gly Gly Gln Arg Gln Arg Val Ser Leu Ala Arg Ala Val Tyr Ser Asp
        755                 760                 765

Ala Asp Ile Phe Leu Leu Asp Asp Pro Leu Ser Ala Val Asp Ser His
    770                 775                 780

Val Ala Lys His Ile Phe Asp His Val Ile Gly Pro Glu Gly Val Leu
785                 790                 795                 800

Ala Gly Lys Thr Arg Val Leu Val Thr His Gly Ile Ser Phe Leu Pro
                805                 810                 815

Gln Thr Asp Phe Ile Ile Val Leu Ala Asp Gly Gln Val Ser Glu Met
            820                 825                 830

Gly Pro Tyr Pro Ala Leu Leu Gln Arg Asn Gly Ser Phe Ala Asn Phe
        835                 840                 845

Leu Cys Asn Tyr Ala Pro Asp Glu Asp Gln Gly His Leu Glu Asp Ser
    850                 855                 860

Trp Thr Ala Leu Glu Gly Ala Glu Asp Lys Glu Ala Leu Leu Ile Glu
865                 870                 875                 880

Asp Thr Leu Ser Asn His Thr Asp Leu Thr Asp Asn Asp Pro Val Thr
                885                 890                 895

Tyr Val Val Gln Lys Gln Phe Met Arg Gln Leu Ser Ala Leu Ser Ser
            900                 905                 910

Asp Gly Glu Gly Gln Gly Arg Pro Val Pro Arg Arg His Leu Gly Pro
        915                 920                 925

Ser Glu Lys Val Gln Val Thr Glu Ala Lys Ala Asp Gly Ala Leu Thr
    930                 935                 940

Gln Glu Glu Lys Ala Ala Ile Gly Thr Val Glu Leu Ser Val Phe Trp
945                 950                 955                 960

Asp Tyr Ala Lys Ala Val Gly Leu Cys Thr Thr Leu Ala Ile Cys Leu
                965                 970                 975

Leu Tyr Val Gly Gln Ser Ala Ala Ala Ile Gly Ala Asn Val Trp Leu
            980                 985                 990

Ser Ala Trp Thr Asn Asp Ala Met  Ala Asp Ser Arg Gln  Asn Asn Thr
        995                 1000                 1005

Ser Leu  Arg Leu Gly Val Tyr  Ala Ala Leu Gly Ile  Leu Gln Gly
    1010                 1015                 1020

Phe Leu  Val Met Leu Ala Ala  Met Ala Met Ala Ala  Gly Gly Ile
    1025                 1030                 1035

Gln Ala  Ala Arg Val Leu His  Gln Ala Leu Leu His  Asn Lys Ile
    1040                 1045                 1050

Arg Ser  Pro Gln Ser Phe Phe  Asp Thr Thr Pro Ser  Gly Arg Ile
    1055                 1060                 1065

Leu Asn  Cys Phe Ser Lys Asp  Ile Tyr Val Val Asp  Glu Val Leu
    1070                 1075                 1080

Ala Pro  Val Ile Leu Met Leu  Leu Asn Ser Phe Phe  Asn Ala Ile
    1085                 1090                 1095

Ser Thr  Leu Val Val Ile Met  Ala Ser Thr Pro Leu  Phe Thr Val
    1100                 1105                 1110

Val Ile Leu Pro Leu Ala Val Leu Tyr Thr Leu Val Gln Arg Phe
    1115                1120                1125

Tyr Ala Ala Thr Ser Arg Gln Leu Lys Arg Leu Glu Ser Val Ser
    1130                1135                1140

Arg Ser Pro Ile Tyr Ser His Phe Ser Glu Thr Val Thr Gly Ala
    1145                1150                1155

Ser Val Ile Arg Ala Tyr Asn Arg Ser Arg Asp Phe Glu Ile Ile
    1160                1165                1170

Ser Asp Thr Lys Val Asp Ala Asn Gln Arg Ser Cys Tyr Pro Tyr
    1175                1180                1185

Ile Ile Ser Asn Arg Trp Leu Ser Ile Gly Val Glu Phe Val Gly
    1190                1195                1200

Asn Cys Val Val Leu Phe Ala Ala Leu Phe Ala Val Ile Gly Arg
    1205                1210                1215

Ser Ser Leu Asn Pro Gly Leu Val Gly Leu Ser Val Ser Tyr Ser
    1220                1225                1230

Leu Gln Val Thr Phe Ala Leu Asn Trp Met Ile Arg Met Met Ser
    1235                1240                1245

Asp Leu Glu Ser Asn Ile Val Ala Val Glu Arg Val Lys Glu Tyr
    1250                1255                1260

Ser Lys Thr Glu Thr Glu Ala Pro Trp Val Val Glu Gly Ser Arg
    1265                1270                1275

Pro Pro Glu Gly Trp Pro Pro Arg Gly Glu Val Glu Phe Arg Asn
    1280                1285                1290

Tyr Ser Val Arg Tyr Arg Pro Gly Leu Asp Leu Val Leu Arg Asp
    1295                1300                1305

Leu Ser Leu His Val His Gly Gly Glu Lys Val Gly Ile Val Gly
    1310                1315                1320

Arg Thr Gly Ala Gly Lys Ser Ser Met Thr Leu Cys Leu Phe Arg
    1325                1330                1335

Ile Leu Glu Ala Ala Lys Gly Glu Ile Arg Ile Asp Gly Leu Asn
    1340                1345                1350

Val Ala Asp Ile Gly Leu His Asp Leu Arg Ser Gln Leu Thr Ile
    1355                1360                1365

Ile Pro Gln Asp Pro Ile Leu Phe Ser Gly Thr Leu Arg Met Asn
    1370                1375                1380

Leu Asp Pro Phe Gly Ser Tyr Ser Glu Glu Asp Ile Trp Trp Ala
    1385                1390                1395

Leu Glu Leu Ser His Leu His Thr Phe Val Ser Ser Gln Pro Ala
    1400                1405                1410

Gly Leu Asp Phe Gln Cys Ser Glu Gly Gly Glu Asn Leu Ser Val
    1415                1420                1425

Gly Gln Arg Gln Leu Val Cys Leu Ala Arg Ala Leu Leu Arg Lys
    1430                1435                1440

Ser Arg Ile Leu Val Leu Asp Glu Ala Thr Ala Ala Ile Asp Leu
    1445                1450                1455

Glu Thr Asp Asn Leu Ile Gln Ala Thr Ile Arg Thr Gln Phe Asp
    1460                1465                1470

Thr Cys Thr Val Leu Thr Ile Ala His Arg Leu Asn Thr Ile Met
    1475                1480                1485

Asp Tyr Thr Arg Val Leu Val Leu Asp Lys Gly Val Val Ala Glu
    1490                1495                1500

Phe Asp Ser Pro Ala Asn Leu Ile Ala Ala Arg Gly Ile Phe Tyr
    1505                1510                1515

Gly Met Ala Arg Asp Ala Gly Leu Ala
    1520                1525

<210> 15
<211> 402
<212> PRT
<213> Homo sapiens

<400> 15

Met Gln Met Ser Pro Ala Leu Thr Cys Leu Val Leu Gly Leu Ala Leu
1               5                   10                  15

Val Phe Gly Glu Gly Ser Ala Val His His Pro Pro Ser Tyr Val Ala
            20                  25                  30

His Leu Ala Ser Asp Phe Gly Val Arg Val Phe Gln Gln Val Ala Gln
        35                  40                  45

Ala Ser Lys Asp Arg Asn Val Val Phe Ser Pro Tyr Gly Val Ala Ser
    50                  55                  60

Val Leu Ala Met Leu Gln Leu Thr Thr Gly Gly Glu Thr Gln Gln Gln
65                  70                  75                  80

Ile Gln Ala Ala Met Gly Phe Lys Ile Asp Asp Lys Gly Met Ala Pro
                85                  90                  95

Ala Leu Arg His Leu Tyr Lys Glu Leu Met Gly Pro Trp Asn Lys Asp
            100                 105                 110

Glu Ile Ser Thr Thr Asp Ala Ile Phe Val Gln Arg Asp Leu Lys Leu
        115                 120                 125

Val Gln Gly Phe Met Pro His Phe Phe Arg Leu Phe Arg Ser Thr Val
    130                 135                 140

Lys Gln Val Asp Phe Ser Glu Val Glu Arg Ala Arg Phe Ile Ile Asn
145                 150                 155                 160

Asp Trp Val Lys Thr His Thr Lys Gly Met Ile Ser Asn Leu Leu Gly
                165                 170                 175

Lys Gly Ala Val Asp Gln Leu Thr Arg Leu Val Leu Val Asn Ala Leu
            180                 185                 190

Tyr Phe Asn Gly Gln Trp Lys Thr Pro Phe Pro Asp Ser Ser Thr His
        195                 200                 205

Arg Arg Leu Phe His Lys Ser Asp Gly Ser Thr Val Ser Val Pro Met
    210                 215                 220

Met Ala Gln Thr Asn Lys Phe Asn Tyr Thr Glu Phe Thr Thr Pro Asp
225                 230                 235                 240

Gly His Tyr Tyr Asp Ile Leu Glu Leu Pro Tyr His Gly Asp Thr Leu
                245                 250                 255

Ser Met Phe Ile Ala Ala Pro Tyr Glu Lys Glu Val Pro Leu Ser Ala
            260                 265                 270

Leu Thr Asn Ile Leu Ser Ala Gln Leu Ile Ser His Trp Lys Gly Asn

275                 280                 285

Met Thr Arg Leu Pro Arg Leu Leu Val Leu Pro Lys Phe Ser Leu Glu
    290                 295                 300

Thr Glu Val Asp Leu Arg Lys Pro Leu Glu Asn Leu Gly Met Thr Asp
305                 310                 315                 320

Met Phe Arg Gln Phe Gln Ala Asp Phe Thr Ser Leu Ser Asp Gln Glu
                325                 330                 335

Pro Leu His Val Ala Gln Ala Leu Gln Lys Val Lys Ile Glu Val Asn
            340                 345                 350

Glu Ser Gly Thr Val Ala Ser Ser Ser Thr Ala Val Ile Val Ser Ala
        355                 360                 365

Arg Met Ala Pro Glu Glu Ile Ile Met Asp Arg Pro Phe Leu Phe Val
    370                 375                 380

Val Arg His Asn Pro Thr Gly Thr Val Leu Phe Met Gly Gln Val Ile
385                 390                 395                 400

Glu Pro

<210>  16
<211>  268
<212>  PRT
<213>  Homo sapiens

<400>  16

<210>  17
<211>  21
<212>  DNA
<213>  Artificial Sequence

<220>
<223>  Small hairping RNA (shRNA) against NAMPT

<400>  17
aagatcctgt tccaggctat t                                                21

<210>  18
<211>  21
<212>  DNA
<213>  Artificial Sequence

<220>
<223>  QIAGEN non-coding sequence

<400>  18
ggaatctcat tcgatgcata c                                                21